# EUROPEAN PATENT SPECIFICATION

(11) **EP 1 687 338 B1**
(45) Date of publication and mention of the grant of the patent: **20.10.2010**
(21) Application number: 03775003.1
(22) Date of filing: 07.11.2003
(51) Int. Cl.: C07K 16/28, A61K 39/395, C12N 15/13, C07K 19/00, A61P 35/00, C07K 16/18, G01N 33/577

(54) **CAMELIDAE SINGLE DOMAIN ANTIBODIES VHH DIRECTED AGAINST EPIDERMAL GROWTH FACTOR RECEPTOR AND USES THEREOF**
CAMELIDAE SCHWERE KETTEN ANTIKÖRPER VHHS GEGEN EPIDERMALEN WACHSTUMFAKTOR REZEPTOR (EGFR) UND IHRE VERWENDUNG
ANTICORPS A DOMAINE UNIQUE VHH DE CAMELIDAE DIRIGES CONTRE LE RECEPTEUR DE FACTEUR DE CROISSANCE EPIDERMIQUE ET UTILISATIONS DE CEUX-CI

(43) Date of publication of application: 09.08.2006
(60) Divisional application: 10172630.5
(73) Proprietor: Ablynx N.V., 9052 Zwijnaarde (BE)
(72) Inventor: LAEREMANS, Toon, B-1653 Dworp (BE); VAN BERGEN EN HENEGOUWEN, Paul P.M.P., 3584 GE Utrecht (NL)
(74) Representative: Taormino, Joseph Paul
(86) International application number: PCT/BE2003/000189
(87) International publication number: WO 2005/044858

(56) References cited:
- US-A1- 2002 058 033
- US-A1- 2003 092 892
- ARBABI GHAHROUDI M ET AL: "SELECTION AND IDENTIFICATION OF SINGLE DOMAIN ANTIBODY FRAGMENTS FROM CAMEL HEAVY-CHAIN ANTIBODIES" FEBS LETTERS, ELSEVIER SCIENCE PUBLISHERS, AMSTERDAM, NL, vol. 414, no. 3, 15 September 1997 (1997-09-15), pages 521-526, XP002069903 ISSN: 0014-5793
- CORTEZ-RETAMOZO V ET AL: "Efficient tumor targeting by single-domain antibody fragments of camels" INTERNATIONAL JOURNAL OF CANCER, NEW YORK, NY, US, vol. 98, no. 3, 20 March 2002 (2002-03-20), pages 456-462, XP002248403 ISSN: 0020-7136
- TANHA J ET AL: "Selection by phage display of llama conventional VH fragments with heavy chain antibody VHH properties" JOURNAL OF IMMUNOLOGICAL METHODS, ELSEVIER, AMSTERDAM, NL, vol. 263, no. 1-2, 1 May 2002 (2002-05-01), pages 97-109, XP004354388 ISSN: 0022-1759

## Description

### FIELD OF THE INVENTION

The present invention provides single domain antibodies according to claim 1. The present invention further relates to their use in diagnosis and therapy. Such antibodies may have a framework sequence with high homology to the human framework sequences. Composition comprising antibodies to epidermal growth factor receptor alone or in combination with other drugs are described.

### BACKGROUND TO THE INVENTION

EGFR is part of the ERBB receptor family, which has four closely related members- EGFR (ERBB1), HER2 (ERBB2), HER3 (ERBB3) and HER4 (ERBB4)- that consist of an extracellular ligand-binding domain, a transmembrane domain and an intracellular tyrosine kinase domain (Yarden et al. 2001, Nature Rev. Mol. Cell Biol. 2, 127-137). The first step in the mitogenic stimulation of epidermal cells is the specific binding of ligands such as epidermal growth factor (EGF) or transforming growth factor alpha (TGFα) to a membrane glycoprotein known as the epidermal growth factor receptor (EGF receptor). (Carpenter et al. 1979, Epidermal Growth Factor, Annual Review Biochem., Vol. 48, 193-216). The EGF receptor is composed of 1,186 amino acids which are divided into an extracellular portion of 621 residues and a cytoplasmic portion of 542 residues connected by a single hydrophobic transmembrane segment of 23 residues. (Ullrich, et al. 1986, Human Epidermal Growth Factor cDNA Sequence and Aberrant Expression of the Amplified Gene in A-431 Epidermoid Carcinoma Cells, Nature, Vol. 309, 418-425). The external portion of the EGF receptor can be subdivided into four domains. It has been demonstrated that domain I and III, flanked by two cysteine rich domains, are likely to contain the EGF binding site of the receptor. (Ogiso et al. 2002. Crystal structure of the complex of human epidermal growth factor and receptor extracellular domains. Cell 110, 775-787. Garrett et al. 2002. Crystal structure of a truncated epidermal growth factor receptor extracellular domain bound to transforming growth factor alpha. Cell 110. 763-773.). The binding of monovalent EGF to domain I and III leads to the initiation of pleiotropic responses leading to DNA synthesis and cell proliferation and differentiation.

Monovalent ligand binding to EGFR causes a conformational change of domain II of the receptor ectodomain, leading to receptor dimerization which activates the tyrosine kinase activity in the intracellular domain. This leads to receptor transphosphorylation and the initiation of a myriad of signal transduction cascades. Activation of EGFR has been implicated in processes involved in tumour growth and progression, including cell proliferation, angiogenesis, metastasis, inhibition of apoptosis and resistance to radio- or chemotherapy. EGFR is expressed in a wide variety of tumours of epithelial origin, including >40% of NSCLC (none-small-cell-lung cancer), >95% of head and neck cancer, >30% of pancreatic cancer, >90% of renal carcinoma, > 35% of ovarian cancer, >40% of glioma and >31% of bladder cancer (Salomon et al. 1995. Crit. Review Oncol. Hematol, 19, 183-232). It seems that high levels of EGFR expression are associated with disease progression, increased metastasis and poor prognosis, providing a strong rationale for developing effective EGFR targeting antibodies for the treatment of various solid tumors. It has been found in various types of human tumor cells that those cells are characterized by a dysregulation of EGF receptor signaling due to receptor overexpression and the presence of constitutively signaling EGFR heterodimers or EGFR mutant forms. Breast cancer cells exhibit a positive correlation between EGF receptor density and tumor size and a negative correlation with the extent of differentiation. (Sainsbury et al. 1985, Epidermal Growth Factor Receptors and Oestrogen Receptors in Human Breast Cancer, Lancet, Vol. 1, 364-366; Sainsbury et al. 1985, Presence of Epidermal Growth Factor Receptor as an Indicator of Poor Prognosis in Patients with Breast Cancer, J. Clin. Path., Vol. 38, 1225-1228; Sainsbury et al. 1987. Epidermal-Growth-Factor Receptor Status as Predictor of Early Recurrence and Death From Breast Cancer, Lancet, Vol. 1, 1398-1400). As synovial fibroblasts and keratinocytes are cell types that also express EGF receptor, these cells are candidate target cells for treatment of inflammatory arthritis and psoriasis, respectively.

EGFR has also been implicated in several other diseases, such as inflammatory arthritis (US 5906820, US 5614488), and hypersecretion of mucus in the lungs (US 6566324, US 6551989).

Many of the EGFR targeting antibodies such as IMC-C225 (Erbitux, Imclone), EMD72000 (Merck Darmstadt), ABX-EGF (Abgenix), h-R3 (theraCIM, YM Biosciences) and Humax-EGFR (Genmab) were isolated as antibodies that prevent binding of ligand to the receptor. Yet none of these antibodies nor the presently available drugs are completely effective for the treatment of cancer, and most are limited by severe toxicity. In addition, it is extremely difficult and a lengthy process to develop a new chemical entity (NCE) with sufficient potency and selectivity to such target sequence. The primary goal in treating tumors is to kill all the cells of the tumor. A therapeutic agent that kills the cell is defined as cytotoxic. A therapeutic agent that merely prevents the cells from replicating rather than killing the cells is defined as cytostatic. Known antibody-based therapeutics which bind to the EGF receptor merely prevent the cells from replicating and thus such conventional antibodies act as a cytostatic agent (EP 667165, EP 359282, US 5844093).

On the other hand antibodies offer significant potential as drugs because they have exquisite specificity to their target and a low inherent toxicity. In addition, the development time can be reduced considerably when compared to the development of new chemical entities (NCE's). However, conventional antibodies are difficult to raise against multimeric proteins where the receptor-binding domain of the ligand is embedded in a groove or at the interphase between the two subunits, as is the case with Epidermal Growth Factor Receptor. Heavy chain antibodies described in the invention which are derived from *Camelidae,* are known to have cavity-binding propensity (WO97/49805; Lauwereys et al, EMBO J. 17, 5312, 1998)). Therefore, such heavy chain antibodies are inherently suited to bind to receptor binding domains of such ligands as EGF and may therefore operate via a different mechanism of action to yield a cytotoxic effect on tumour cells. In addition, such antibodies are known to be stable over long periods of time, therefore increasing their shelf-life (Perez et al, Biochemistry, 40, 74, 2001). Furthermore, such heavy chain antibody fragments can be produced 'en-masse' in fermentors using cheap expression systems compared to mammalian cell culture fermentation, such as yeast or other microorganisms (EP 0 698 097).

The use of antibodies derived from sources such as mouse, sheep, goat, rabbit etc., and humanized derivatives thereof as a treatment for conditions which require a cytostatic or cytotoxic effect on tumor cells is problematic for several reasons. Traditional antibodies are not stable at room temperature, and have to be refrigerated for preparation and storage, requiring necessary refrigerated laboratory equipment, storage and transport, which contribute towards time consumption and expense. Refrigeration is sometimes not feasible in developing countries. Furthermore, the manufacture or small-scale production of said antibodies is expensive because the mammalian cellular systems necessary for the expression of intact and active antibodies require high levels of support in terms of time and equipment, and yields are very low. Furthermore the large size of conventional antibodies, would restrict tissue penetration, for example, at the site of a solid tumor. Furthermore, traditional antibodies have a binding activity which depends upon pH, and hence are unsuitable for use in environments outside the usual physiological pH range such as, for example, in treating colorectal cancer. Furthermore, traditional antibodies are unstable at low or high pH and hence are not suitable for oral administration. However, it has been demonstrated that *Camelidae* antibodies resist harsh conditions, such as extreme pH, denaturing reagents and high temperatures (Ewert S et al, Biochemistry 2002 Mar 19; 41(11):3628-36), so making them suitable for delivery by oral administration. Furthermore, traditional antibodies have a binding activity, which depends upon temperature, and hence are unsuitable for use in assays or kits performed at temperatures outside biologically active-temperature ranges (e.g. 37 ± 20°C).

Polypeptide therapeutics and in particular antibody-based therapeutics have significant potential as drugs because they have exquisite specificity to their target and a low inherent toxicity. However, it is known by the skilled addressee that an antibody which has been obtained for a therapeutically useful target requires additional modification in order to prepare it for human therapy, so as to avoid an unwanted immunological reaction in a human individual upon administration thereto. The modification process is commonly termed "humanisation". It is known by the skilled artisan that antibodies raised in species, other than in humans, require humanisation to render the antibody therapeutically useful in humans ( (1) CDR grafting : Protein Design Labs: US 6180370, US 5693761; Genentech US 6054297; Celltech: 460167, EP 626390, US 5859205; (2) Veneering: Xoma: US 5869619, US 5766886, US 5821123). There is a need for a method for producing antibodies which avoids the requirement for substantial humanisation, or wh ich completely obviates the need for humanisation. There is a need for a new class of antibodies which have defined framework regions or amino acid residues and which can be administered to a human subject without the requirement for substantial humanisation, or the need for humanisation at all.

Another important drawback of conventional antibodies is that they are complex, large molecules and therefore relatively unstable, and they are sensitive to breakdown by proteases. This means that conventional antibody drugs cannot be administered orally, sublingually, topically, nasally, vaginally, rectally or by inhalation because they are not resistant to the low pH at these sites, the action of proteases at these sites and in the blood and/or because of their large size. They have to be administered by injection (intravenously, subcutaneously, etc.) to overcome some of these problems. Administration by injection requires specialist training in order to use a hypodermic syringe or needle correctly and safely. It further requires sterile equipment, a liquid formulation of the therapeutic polypeptide, vial packing of said polypeptide in a sterile and stable form and, of the subject, a suitable site for entry of the needle. Furthermore, subjects commonly experience physical and psychological stress prior to and upon receiving an injection. Therefore, there is need for a method for the delivery of therapeutic polypeptides which avoids the need for injection which is not only cost/time saving, but which would also be more convenient and more comfortable for the subject.

### THE AIMS OF THE PRESENT INVENTION

It is an aim of the present invention to provide polypeptides comprising one or more single domain antibodies which bind to EGFR, homologues of said polypeptides, functional portions of homologues . Said polypeptides can i) inhibit binding of the natural ligand to the receptor and /or, ii) prevent homo- and heterodimerization of the receptor and/or iii) induce apoptosis in human cells, thereby modifying the biological activity of Epidermal Growth Factor Receptor upon binding. Such polypeptides might bind into the ligand-binding groove of Epidermal Growth Factor Receptor, or might not bind in the ligand binding groove. Such polypeptides are single domain antibodies.

It is a further aim of the present invention to provide single domain antibodies which may be heavy chain antibodies, antibodies naturally devoid of light chains, single domain antibodies derived from conventional 4-chain antibodies, engineered antibodies and single domain scaffolds other than those derived from antibodies.

It is a further aim of the invention to provide a method of administering anti- Epidermal Growth Factor Receptor polypeptides intravenously orally, sublingually, topically, nasally, vaginally, rectally or by inhalation.

### SUMMARY OF THE INVENTION

In one embodiment, the present invention relates to an anti-EGFR polypeptide comprising at least one single domain antibody directed against EGFR, wherein said single domain antibody is derived from a heavy chain antibody naturally devoid of light chain (VHH) and wherein said at least one single domain antibody is able to internalize EGFR⁺ cells, in particular to said anti-EGFR polypeptide obtainable by incubating recombinant phages expressing a repertoir of cloned VHHs with EGFR⁺ cells under conditions to allow internalization of the phages and subsequent release of the internalized phages. The EGFR⁺ cell may be selected from HER-14 and A431 cells.

More specifically, the present invention provides anti-EGFR polypeptides as discussed above, wherein the at least one single domain antibody corresponds to a sequence represented by any of SEQ ID NOs: 21, 22 or
a. an homologous sequence of any of SEQ ID NOs: 21, 22 with a sequence identity of more than 70%, more than 80% or more than 90% with the parent sequence;
b. a functional portion of any of SEQ ID NOs: 21, 22 that maintains the interaction with the target with affinity of 1 x 10⁻⁶ M or better;
c. a functional portion of any of SEQ ID NOs: 21, 22 that comprises a partial deletion of the complete amino acid sequence and still maintains the binding site(s) and protein domain(s) necessary for the binding of and interaction with EGFR.

The invention more particularly provides anti-EGFR polypeptides as described above, wherein the at least one single domain antibody competes for or inhibits binding of the natural ligand to EGFR. The natural ligand may be EGF.

In one embodiment, the anti-EGFR polypeptide as described above comprises at least one single domain antibody which is capable of binding to its target with an affinity of better than 10⁻⁶ M.

In a further embodiment, the anti-EGFR polypeptide as described above may further comprise at least one single domain antibody directed against one or more serum proteins. The serum proteins may be any of serum albumin, serum immunoglobulins, thyroxine-binding protein, transferrin, or fibrinogen or a fragment thereof.

In one embodiment, the anti-EGFR polypeptide as described above comprises at least one single domain antibody which is a VHH domain. The said VHH domain may be carrying an amino acid from the group consisting of glycine, alanine, valine, leucine, isoleucine, proline, phenylalanine, tyrosine, tryptophan, methionine, serine, threonine, asparagine, or glutamine at position 45 according to the Kabat numbering; or it may be carrying an arginine residue at position 103 according to the Kabat numbering.

In a particular embodiment, the anti-EGFR polypeptide as described above comprises a VHH domain, in which said VHH domain is obtained by immunizing a camel and obtaining hybridoma's therefrom, or by cloning a library of single domain antibodies subsequent selection by using phage display.

In a further embodiment, the at least one single domain antibody is a humanized VHH. It may be humanized by replacing one or more of the *Camelidae* amino acids by their human counterpart as found in the human consensus sequence. More specifically, it may be humanized by replacing any of the following residues either alone or in combination: FR1 positions 1, 5, 28 and 30, the hallmark amino acid at position 44 and 45 in FR2, FR3 residues 74, 75, 76, 83, 84, 93 and 94 and positions 103, 104, 108 and 111 in FR4; numbering according to the Kabat numbering.

In a further embodiment, the anti-EGFR polypeptide as described above may further comprise at least one single domain antibody selected from the group consisting of anti-IFN-gamma single domain antibody, anti-TNF-alpha single domain antibody, anti-TNF-alpha receptor single domain antibody and anti-IFN-gamma receptor single domain antibody.

The invention provides an anti-EGFR polypeptide as described above, wherein the number of single domain antibodies directed against EGFR is at least two. The two or more single domain antibodies may be different in sequence, or identical in sequence. The two or more single domain antibodies may be fused genetically at the DNA level. The single domain antibodies may be linked to each other directly. The anti-EGFR polypeptide may be a bivalent, trivalent or tetravalent molecule.

The invention further provides anti-EGFR polypeptide as described above, further comprising a carrier that enhances the transfer through the intestinal wall into the bloodstream. The carrier may be a VHH that binds specifically to a receptor on the intestinal wall.

The invention also provides anti-EGFR polypeptide as described above, further comprising a carrier that enhances transport of the polypeptide from the lung lumen to the blood. The carrier may be a VHH that binds specifically to a receptor on the mucosal surface. The said receptor may be the Fc receptor N (FcRn).

The invention also provides nucleic acid encoding a polypeptide as described above, as well as a host cell comprising the nucleic acid.

In a further aspect, the invention provides a composition comprising a polypeptide as described above, or a nucleic acid as described above, and a suitable pharmaceutical vehicle. The composition may be adapted to the chosen route of administration, *i*.*e*., orally, vaginally, rectally, parenterally, intra-nasally, by inhalation, sublingually, intravenous, intramuscular, topical or subcutaneous routes, specifically, it may be suitable for injection or infusion.

The invention also provides a therapeutic composition comprising an anti-EGFR polypeptide in combination with anti-neoplastic or chemotherapeutic agent, which in one embodiment may be for separate administration of the components.

The invention further provides the anti-EGFR polypeptide, nucleic acid, or composition as described above for use in the treating, preventing and/or alleviating the symptoms of disorders relating to inflammatory processes, or having cytostatic or cytotoxic effects on tumors;
or for use in the treating, preventing and/or alleviating epithelial cancers, such as lung, liver, central nervous system, bone, blood and lymphatic system, colon, breast, prostate, rectum, bladder, head and neck, ovarian, testis, pancreatic and squamos cell carcinoma,
or for use in the treating, preventing and/or alleviating autoimmune diseases, such as Addison's disease (adrenal), Autoimmune diseases of the ear (ear), Autoimmune diseases of the eye (eye), Autoimmune hepatitis (liver), Autoimmune parotitis (parotid glands), Crohn's disease (intestine), Diabetes Type I (pancreas), Epididymitis (epididymis), Glomerulonephritis (kidneys), Graves' disease (thyroid), Guillain-Barre syndrome (nerve cells), Hashimoto's disease (thyroid), Hemolytic anemia (red blood cells), Systemic lupus erythematosus (multiple tissues), Male infertility (sperm), Multiple sclerosis (nerve cells), Myasthenia Gravis (neuromuscular junction), Pemphigus (primarily skin), Psoriasis (skin), Rheumatic fever (heart and joints), Rheumatoid arthritis (joint lining), Sarcoidosis (multiple tissues and organs), Scleroderma (skin and connective tissues), Sjogren's syndrome (exocrine glands, and other tissues), Spondyloarthropathies (axial skeleton, and other tissues), Thyroiditis (thyroid), Vasculitis (blood vessels).

More specifically the invention provides the use of a polypeptide or polypeptide construct or of a nucleic acid as defined above, for the preparation of a medicament for treating a disorders relating to inflammatory processes and cancer,
or for the preparation of a medicament for the treating conditions which require blocking of EGFR action, blocking of ligand binding to EGFR, prevention of EGFR dimerization, and/or induction of apoptosis,
or for the preparation of a medicament for the treating, preventing and/or alleviating epithelial cancers, such as lung, liver, central nervous system, bone, blood and lymphatic system, colon, breast, prostate, rectum, bladder, head and neck, ovarian, testis, pancreatic and squamos cell carcinoma,
or for the preparation of a medicament for the treating, preventing and/or alleviating autoimmune diseases, such as Addison's disease (adrenal), Autoimmune diseases of the ear (ear), Autoimmune diseases of the eye (eye), Autoimmune hepatitis (liver), Autoimmune parotitis (parotid glands), Crohn's disease (intestine), Diabetes Type I (pancreas), Epididymitis (epididymis), Glomerulonephritis (kidneys), Graves' disease (thyroid), Guillain-Barre syndrome (nerve cells), Hashimoto's disease (thyroid), Hemolytic anemia (red blood cells), Systemic lupus erythematosus (multiple tissues), Male infertility (sperm), Multiple sclerosis (nerve cells), Myasthenia Gravis (neuromuscular junction), Pemphigus (primarily skin), Psoriasis (skin), Rheumatic fever (heart and joints), Rheumatoid arthritis (joint lining), Sarcoidosis (multiple tissues and organs), Scleroderma (skin and connective tissues), Sjogren's syndrome (exocrine glands, and other tissues), Spondyloarthropathies (axial skeleton, and other tissues), Thyroiditis (thyroid), Vasculitis (blood vessels).

The invention further provides a method of producing a polypeptide as described above, comprising: culturing host cells comprising nucleic acid capable of encoding the said polypeptide or polypeptide construct under conditions allowing the expression of the polypeptide, and recovering the produced polypeptide from the culture. The method may involve host cells which are bacterial cells, yeast cells, mammalian cells or insect cells, more specifically bacterial cells or yeast cells, in particular *E. coli* cells, *S. cerevisiae* cells or *P. pastoris* cells.

Furthermore, the invention also provides a method of diagnosing a disorder characterised by the dysfunction of EGFR comprising the steps of: contacting a sample with a polypeptide as described above, detecting binding of said polypeptide to said sample, and comparing the binding detected in step (b) with a standard, wherein a difference in binding relative to said sample is diagnostic of a disorder characterised by dysfunction of EGFR.

The invention also provides a kit for screening for a disorder characterised by dysfunction of EGFR comprising a polypeptide according to the invention.

### BRIEF DESCRIPTION OF FIGURES AND TABLES

**Figure 1****.** ELISA to detect A431 specific antibody titers in llama serum.
**Figure 2****.** Detection of EGFR specific antibody titers in llama serum.
**Figure 3****.** Detection of EGFR specific antibody titers in serum of llama 024 and025 (panel A) and of llama 026 and 027 (panel B).
**Figure 4**. Phage response to EGFR.
**Figure 5****.** Amino acid alignment of 31 clones identified by the epitope specific elution selection procedure.
**Figure 6****.** Phage ELISA on cells (panel A) or on solid-phase immobilized EGFR (panel B) of the 20 unique EGFR specific clones identified via the epitope specific elution selection procedure.
**Figure 7****.** Internalization of EGFR-IIIa42 with Her-14 (panel A) and 3T3 (panel B).
**Table 1.** Immunization schedule and tissue collections.
**Table 2.** Overview of constructed libraries.
**Table 3.** Overview of epitope specific elution selection procedure.
**Table 4.** Overview of 'internalization' selection procedure.
**Table 5.** EGFR specific antibody variable domain amino acid sequences.
**Table 6.** Primer identification table.

### DETAILED DESCRIPTION

The present invention relates to an anti-Epidermal Growth Factor Receptor (EGFR) polypeptide, comprising at least one single domain antibody according to claim 1 which is directed towards Epidermal Growth Factor Receptor. The invention also relates to nucleic acids capable of encoding said polypeptides.

Another embodiment of the present invention is an anti-Epidermal Growth Factor Receptor polypeptide wherein at least one single domain antibody corresponds to a sequence corresponding to any of SEQ ID NOs: 21 or 22 as shown in Table 5. Said sequences are derived from *Camelidae* heavy chain antibodies (VHHs) which are directed towards Epidermal Growth Factor Receptor.

Single domain antibodies are antibodies whose complementary determining regions are part of a single domain polypeptide. According to the invention, a single domain antibodies as used herein is a naturally occurring single domain antibody known as heavy chain antibody devoid of light chains. Such single domain antibodies are disclosed in WO 94/04678 for example. For clarity reasons, this variable domain derived from a heavy chain antibody naturally devoid of light chain is known herein as a *VHH* or *nanobody* to distinguish it from the conventional VH of four chain immunoglobulins. Such a VHH molecule can be derived from antibodies raised in *Camelidae* species, for example in camel, dromedary, llama, vicuña, alpaca and guanaco. Other species besides *Camelidae* may produce heavy chain antibodies naturally devoid of light chain; such VHHs are within the scope of the invention.

VHHs, according to the present invention, and as known to the skilled addressee are heavy chain variable domains derived from immunoglobulins naturally devoid of light chains such as those derived from *Camelidae* as described in WO 94/04678 (and referred to hereinafter as VHH domains or nanobodies). VHH molecules are about 10x smaller than IgG molecules. They are single polypeptides and very stable, resisting extreme pH and temperature conditions. Moreover, they are resistant to the action of proteases which is not the case for conventional antibodies. Furthermore, *in vitro* expression of VHHs produces high yield, properly folded functional VHHs. In addition, antibodies generated in *Camelids* will recognize epitopes other than those recognised by antibodies generated *in vitro* through the use of antibody libraries or via immunisation of mammals other than *Camelids* (WO 9749805). As such, anti EGFR VHH's may Interact more efficiently with EGFR than conventional antibodies, thereby blocking its interaction with the EGFR ligand(s) more efficiently. Sine VHH's are known to bind into 'unusual' epitopes such as cavities or grooves (WO 97/49805), the affinity of such VHH's may be more suitable for therapeutic treatment.

Another embodiment of the present Invention is an anti-Epidermal Growth Factor Receptor polypeptide according to claim 1 consisting of a sequence corresponding to that of a *Camelidae* VHH directed towards EGFR or a closely related family member. The invention also relates to a homologous sequence, a function portion or a functional portion of a homologous sequence of said polypeptide. The invention also relates to nucleic acids capable of encoding said polypeptides.

A single domain antibody according to claim 1 of the present invention is directed against EGFR or a closely related family member.

EGFR is a principal target according to the invention. According to the invention, as and discussed below, a polypeptide construct may further comprise single domain antibodies directed against other targets such as, for example, serum albumin. A single domain antibody directed against a target means a single domain antibody that is capable of binding to said target with an affinity of better than 10⁻⁶M.

Targets may also be fragments of said targets. Thus a target is also a fragment of said target, capable of eliciting an immune response. A target is also a fragment of said target, capable of binding to a single domain antibody raised against the full length target.

A fragment as used herein refers to less than 100% of the sequence (e.g., 99%, 90%, 80%, 70%, 60%, 50%, 40%, 30%, 20%, 10% etc.), but comprising 5, 6, 7, 8, 9, 10, 12, 13, 14, 15, 16, 17, 18, 19, 20, 21, 22, 23, 24, 25 or more amino acids. A fragment is of sufficient length such that the interaction of interest is maintained with affinity of 1 x 10⁻⁶ M or better.

A fragment as used herein also refers to optional insertions, deletions and substitutions of one or more amino acids which do not substantially alter the ability of the target to bind to a single domain antibody raised against the wild-type target. The number of amino acid insertions deletions or substitutions is preferably up to 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 88, 69 or 70 amino acids.

The present invention further relates to an anti-Epidermal Growth Factor Receptor polypeptide according to claim 1 wherein a single domain antibodies is a VHH belonging to a class having human-like sequences.

One such class is **characterized in that** the VHHs carry an amino acid from the group consisting of glycine, alanine, valine, leucine, isoleucine, proline, phenylalanine, tyrosine, tryptophan, methionine, serine, threonine, asparagine, or glutamine at position 45, such as, for example, L45 and a tryptophan at position 103, according to the Kabat numbering. Such a humanlike sequence is represented by SEQ ID No. 13. As such, polypeptides belonging to this class show a high amino acid sequence homology to human VH framework regions and said polypeptides might be administered to a human directly without expectation of an unwanted immune response therefrom, and without the burden of further humanisation.

Another human-like class of *Camelidae* single domain antibodies has been described in WO 03/035694 and contain the hydrophobic FR2 residues typically found in conventional antibodies of human origin or from other species, but compensating this loss in hydrophilicity by the charged arginine residue on position 103 that substitutes the conserved tryptophan residue present in VH from double-chain antibodies. As such, peptides belonging to these two classes show a high amino acid sequence homology to human VH framework regions and said peptides might be administered to a human directly without expectation of an unwanted immune response therefrom, and without the burden of further humanization. The invention also relates to nucleic acids capable of encoding said polypeptides.

SEQ ID NO: 13 display more than 90% amino add sequence homology to human VH framework regions and therefore said VHH might be administered to patients directly without expectation of an immune response therefrom, and without the additional burden of humanization. Therefore, one aspect of the present invention allows for the direct administration of the polypeptide comprising SEQ ID NO: 13.

Any of the anti- Epidermal Growth Factor Receptor VHHs disclosed herein may be of the traditional class or of a class of human-like *Camelidae* antibodies. Said antibodies may be directed against whole Epidermal Growth Factor Receptor or a fragment thereof, or a fragment of a homologous sequence thereof. These polypeptides include the full length *Camelidae* antibodies, namely Fc and VHH domains.

Anti-albumin VHH's may interact in a more efficient way with serum albumin which is known to be a carrier protein. As a carrier protein some of the epitopes of serum albumin may be inaccessible by bound proteins, peptides and small chemical compounds. Since VHH's are known to bind into 'unusual' or non-conventional epitopes such as cavities (WO 97/49805), the affinity of such VHH's to circulating albumin may be more suitable for therapeutic treatment.

The present invention therefore relates to the finding that an anti-EGFR polypeptide of the invention further comprising one or more single domain antibodies directed against one or more serum proteins of a subject, which surprisingly has significantly prolonged half-life in the circulation of said subject compared with the half-life of the anti-target VHH when not part of said anti-EGFR polypeptide.

Another embodiment of the present invention is an anti-EGFR polypeptide according to claim 1 further comprising at least one single domain antibody directed against a serum protein, said anti-EGFR polypeptide comprising a sequence corresponding to any represented by SEQ ID NOs: 27 to 40 (Table 5).

Another embodiment of the present invention is an anti-EGFR potypeptide according to claim 1, wherein at least one anti-serum protein single domain antibody corresponds to a sequence represented by any of SEQ ID NOs: 23 to 26 and 41 to 53 as shown in Table 5

The serum protein may be any suitable protein found in the serum of subject, or fragment thereof. In one aspect of the invention, the serum protein is serum albumin, serum immunoglobulins, thyroxine-binding protein, transferrin, or fibrinogen. Depending on the intended use such as the required half-life for effective treatment and/or compartimentalisation of the target antigen, the VHH-partner can be directed to one of the above serum proteins.

Furthermore, the said constructs were found to exhibit the same favourable properties of VHHs such as high stability remaining intact In mice, extreme pH resistance, high temperature stability and high target affinity.

Another embodiment of the present invention is a multivalent anti-Epidermal Growth Factor Receptor polypeptide as disclosed herein comprising at least two single domain antibodies according to claim 1 directed against Epidermal Growth Factor Receptor. Such multivalent anti-Epidermal Growth Factor Receptor polypeptides have the advantage of unusually high functional affinity for the target, displaying much higher than expected inhibitory properties compared to their monovalent counterparts.

The multivalent anti-Epidermal Growth Factor Receptor polypeptides have functional affinities that are several orders of magnitude higher than the monovalent parent anti-Epidermal Growth Factor Receptor polypeptides. The inventors have found that the functional affinities of these multivalent polypeptides are much higher than those reported in the prior art for bivalent and multivalent antibodies. Surprisingly, anti- Epidermal Growth Factor Receptor polypeptides of the present invention linked to each other directly or via a short linker sequence show the high functional affinities expected theoretically with multivalent conventional four-chain antibodies.

The inventors have found that such large increased functional activities can be detected preferably with antigens composed of multidomain and multimeric proteins, either in straight binding assays or in functional assays, e.g. cytotoxicity assays.

A multivalent anti-Epidermal Growth Factor Receptor polypeptide as used herein refers to a polypeptide comprising two or more anti- Epidermal Growth Factor Receptor polypeptides according to claim 1 which have been covalently linked. The anti- Epidermal Growth Factor Receptor polypeptides may be identical in sequence or may be different in sequence, but are directed against the same target or antigen. Depending on the number of anti-Epidermal Growth Factor Receptor polypeptides linked, a multivalent anti- Epidermal Growth Factor Receptor polypeptide may be bivalent (2 anti- Epidermal Growth Factor Receptor polypeptides), trivalent (3 anti- Epidermal Growth Factor Receptor polypeptides), tetravalent (4 anti- Epidermal Growth Factor Receptor polypeptides) or have a higher valency molecules.

According to one aspect of the present invention, the anti- Epidermal Growth Factor Receptor polypeptides are linked to each other directly, without use of a linker. According to another aspect of the present invention, the anti- Epidermal Growth Factor Receptor polypeptides are linked to each other via a peptide linker sequence. Such linker sequence may be a naturally occurring sequence or a non-naturally occurring sequence. The linker sequence is expected to be non-immunogenic in the subject to which the anti- Epidermal Growth Factor Receptor polypeptides is administered. The linker sequence may provide sufficient flexibility to the multivalent anti- Epidermal Growth Factor Receptor polypeptide, at the same time being resistant to proteolytic degradation. A non-limiting example of a linker sequences is one that can be derived from the hinge region of VHHs described in WO 96/34103.

It is an aspect of the invention that a multivalent anti- Epidermal Growth Factor Receptor polypeptides disclosed above may be used instead of or as well as the single unit anti-Epidermal Growth Factor Receptor polypeptides in the therapies and methods of delivery as mentioned herein.

The single domain antibodies may be joined to form any of the anti-Epidermal Growth Factor Receptor polypeptides disclosed herein comprising more than one single domain antibody using methods known in the art or any future method. They may be joined non-covalently (e.g. using streptavidin/biotin combination, antibody/tag combination) or covalently. They may be fused by chemical cross-linking by reacting amino acid residues with an organic derivatising agent such as described by Blattler et al, Biochemistry 24,1517-1524; EP294703. Alternatively, the single domain antibody may be fused genetically at the DNA level *i*.*e*. anti-Epidermal Growth Factor Receptor polypeptide formed which encodes the complete polypeptide comprising one or more anti-Epidermal Growth Factor Receptor single domain antibodies. A method for producing bivalent or multivalent anti-Epidermal Growth Factor Receptor polypeptide is disclosed in PCT patent application WO 96/34103. One way of joining VHH antibodies is via the genetic route by linking a VHH antibody coding sequences either directly or via a peptide linker. For example, the C-terminal end of the VHH antibody may be linked to the N-terminal end of the next single domain antibody.

This linking mode can be extended in order to link additional single domain antibodies for the construction and production of tri-, tetra-, etc. functional constructs.

According to one aspect of the present invention, the single domain antibodies are linked to each other via a peptide linker sequence. Such linker sequence may be a naturally occurring sequence or a non-naturally occurring sequence. The linker sequence is expected to be non-immunogenic in the subject to which the anti-IFN-gamma polypeptide is administered. The linker sequence may provide sufficient flexibility to the anti-Epidermal Growth Factor Receptor polypeptide, at the same time being resistant to proteolytic degradation. A non-limiting example of a linker sequences is one that can be derived from the hinge region of VHHs described in WO 96/34103.

The polypeptide disclosed herein may be made by the skilled artisan according to methods known in the art or any future method. For example, VHHs may be obtained using methods known in the art such as by immunizing a camel and obtaining hybridomas therefrom, or by cloning a library of single domain antibodies using molecular biology techniques known in the art and subsequent selection by ELISA with individual clones of unselected libraries or by using phage display.

According to an aspect of the invention an anti-Epidermal Growth Factor Receptor polypeptide may be a homologous sequence of a full-length anti-Epidermal Growth Factor Receptor polypeptide according to claim 1. According to another aspect of the invention, an anti-Epidermal Growth Factor Receptor polypeptide may be a functional portion of a full-length anti-Epidermal Growth Factor Receptor polypeptide according to claim 1. According to another aspect of the invention, an anti-Epidermal Growth Factor Receptor polypeptide may be a functional portion of a homologous sequence of a full length anti-Epidermal Growth Factor Receptor polypeptide according to claim 1. According to an aspect of the invention an anti-Epidermal Growth Factor Receptor polypeptide may comprise a sequence of an anti-Epidermal Growth Factor Receptor polypeptide according to claim 1.

According to an aspect of the invention a single domain antibody according to claim 1 used to form an anti-Epidermal Growth Factor Receptor polypeptide may be a complete single domain antibody (e.g. a VHH) or a homologous sequence thereof. According to another aspect of the invention, a single domain antibody according to claim 1 used to form an anti-Epidermal Growth Factor Receptor polypeptide may be a functional portion of a complete single domain antibody. According to another aspect of the invention, a single domain antibody according to claim 1 used to form an anti-Epidermal Growth Factor Receptor polypeptide may be a homologous sequence of a complete single domain antibody. According to another aspect of the invention, a single domain antibody according to claim 1 used to form an anti-Epidermal Growth Factor Receptor polypeptide may be a functional portion of a homologous sequence of a complete single domain antibody.

As used herein, a homologous sequence of the present invention may comprise additions, deletions or substitutions of one or more amino acids, which do not substantially alter the functional characteristics of the polypeptides of the invention. For the anti-Epidermal Growth Factor Receptor polypeptides, the number of amino acid deletions or substitutions is preferably up to 1, 2, 3, 4, 5, 6, 7, 8, 9, 10, 11, 12, 13, 14, 15, 16,17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69 or 70 amino acids.

A homologous sequence according to the present invention may be a sequence modified by the addition, deletion or substitution of amino acids, said modification not substantially altering the functional characteristics compared with the unmodified polypeptide.

A homologous sequence according to the present invention may be a sequence which exists in other *Camelidae* species such as, for example, camel, dromedary, Ilama, vicuña, alpaca and guanaco.

Where homologous sequence indicates sequence identity, it means a sequence which presents a high sequence identity (more than 70%, 75%, 80%, 85%, 90%, 95% or 98% sequence identity) with the parent sequence and is preferably characterised by similar properties of the parent sequence, namely affinity, said identity calculated using known methods.

Alternatively, a homologous sequence may also be any amino acid sequence resulting from allowed substitutions at any number of positions of the parent sequence according to the formula below:
Ser substituted by Ser, Thr, Gly, and Asn;
Arg substituted by one of Arg, His, Gln, Lys, and Glu;
Leu substituted by one of Leu, Ile, Phe, Tyr, Met, and Val;
Pro substituted by one of Pro, Gly, Ala, and Thr;
Thr substituted by one of Thr, Pro, Ser, Ala, Gly, His, and Gln;
Ala substituted by one of Ala, Gly, Thr, and Pro;
Val substituted by one of Val, Met, Tyr, Phe, Ile, and Leu;
Gly substituted by one of Gly, Ala, Thr, Pro, and Ser;
Ile substituted by one of Ile, Met, Tyr, Phe, Val, and Leu;
Phe substituted by one of Phe, Trp, Met, Tyr, Ile, Val, and Leu;
Tyr substituted by one of Tyr, Trp, Met, Phe, Ile, Val, and Leu;
His substituted by one of His, Glu, Lys, Gln, Thr, and Arg;
Gln substituted by one of Gln, Glu, Lys, Asn, His, Thr, and Arg;
Asn substituted by one of Asn, Glu, Asp, Gin, and Ser;
Lys substituted by one of Lys, Glu, Gin, His, and Arg;
Asp substituted by one of Asp, Glu, and Asn;
Glu substituted by one of Glu, Asp, Lys, Asn, Gin, His, and Arg;
Met substituted by one of Met, Phe, Ile, Val, Leu, and Tyr.

A homologous nucleotide sequence according to the present invention may refer to nucleotide sequences of more than 50, 100, 200, 300, 400, 500, 600, 800 or 1000 nucleotides able to hybridize to the reverse-complement of the nucleotide sequence capable of encoding the patent sequence, under stringent hybridization conditions (such as the ones described by Sambrook et al., Molecular Cloning, Laboratory Manuel, Cold Spring, Harbor Laboratory press, New York.

As used herein, a functional portion refers to a sequence of a single domain antibody that is of sufficient size such that the interaction of interest is maintained with affinity of 1 x 10⁻⁶ M or better.

Alternatively, a functional portion comprises a partial deletion of the complete amino acid sequence and which still maintains the binding site(s) and protein domain(s) necessary for the binding of and interaction with Epidermal Growth Factor Receptor.

As used herein, a functional portion as it refers to the polypeptide sequence an anti-Epidermal Growth Factor Receptor polypeptide refers to less than 100% of the sequence (e.g., 99%, 90%, 80%, 70%, 60% 50% etc.), but comprising 5 or more amino acids or 15 or more nucleotides.

A portion as it refers to the polypeptide of an anti-Epidermal Growth Factor Receptor polypeptide, refers to less than 100% of the sequence (e.g., 99%, 90%, 80%, 70%, 60% 50% etc.), but comprising 5 or more amino acids or 15 or more nucleotides.

One embodiment of the present invention relates to a method for preparing modified polypeptides based upon Ilama antibodies by determining the amino acid residues of the antibody variable domain (VHH) which may be modified without diminishing the native affinity of the domain for antigen and while reducing its immunogenicity with respect to a heterologous species; the use of VHHs having modifications at the identified residues which are useful for administration to heterologous species; and to the VHH so modified. More specifically, the invention relates to the preparation of modified VHHs, which are modified for administration to humans, the resulting VHH themselves, and the use of such "humanized" VHHs in the treatment of diseases in humans. By humanized is meant mutated so that immunogenicity upon administration in human patients is minor or nonexistent. Humanizing a polypeptide, according to the present invention, comprises a step of replacing one or more of the *Camelidae* amino acids by their human counterpart as found in the human consensus sequence, without that polypeptide losing its typical character, *i*.*e*. the humanization does not significantly affect the antigen binding capacity of the resulting polypeptide. Such methods are known by the skilled addressee. Humanization of *Camelidae* single domain antibodies requires the introduction and mutagenesis of a limited amount of amino acids in a single polypeptide chain. This is in contrast to humanization of scFv, Fab', (Fab')2 and IgG, which requires the introduction of amino acid changes in two chains, the light and the heavy chain and the preservation of the assembly of both chains.

As a non-limited example, the polypeptide of SEQ ID 13 containing human-like residues in FR2 was humanized. Humanization required mutagenesis of residues in FR1 at position 1 and 5 which were introduced by the primer used for repertoire cloning and do not occur naturally in the Ilama sequence. Mutagenesis of those residues did not result in loss of binding and/or inhibition activity. Humanization also required mutagenesis of residues in FR3 at position 74, 76, 83, 84, 93. Mutagenesis of those residues did not result in a dramatic loss of binding and/or inhibition activity (data not shown). Combining the mutations of FR1 and FR3 therefore did not affect the binding and/or inhibition activity (data not shown).

Humanization also required mutagenesis of residues in FR4 at position 108. Mutagenesis of Q108L resulted in lower production level in *Escherichia coli.* Position 108 is solvent exposed in camelid VHH, while in human antibodies this position is buried at the VH-VL interface (Spinelli, 1996; Nieba, 1997). In isolated VHHs position 108 is solvent exposed. The introduction of a non-polar hydrophobic Leu instead of polar uncharged Gln can have a drastic effect on the intrinsic folding/stability of the molecule.

As a non-limited example, the polypeptide represented in SEQ ID 6 containing camelid hallmark residues at position 37, 44, 45 and 47 with hydrophilic characteristics was humanized. Replacement of the hydrophilic residues by human hydrophobic residues at positions 44 and 45 (E44G and R45L), did not have an effect on binding and/or inhibition. However, loss of binding and/or inhibition activity was observed when F37V and F47W were introduced. Modeling data confirmed the critical residue 37 to preserve the integrity of the CDR3 loop conformation and hence on activity (data not shown; all numbering according to Kabat).

One embodiment of the present invention is a method for humanizing a VHH comprising the steps of replacing of any of the following residues either alone or in combination:
FR1 position 1, 5, 28 and 30,
the hallmark amino acid at position 44 and 45 in FR2,
FR3 residues 74, 75, 76, 83, 84, 93 and 94 ,
and positions 103, 104, 108 and 111 in FR4 ;
(numbering according to the Kabat numbering).

One embodiment of the present invention is an anti-Epidermal Growth Factor Receptor polypeptide as disclosed herein, or a nucleic acid capable of encoding said polypeptide for use in treating, preventing and/or alleviating the symptoms of disorders relating to inflammatory processes, or having cytostatic or cytotoxic effects on tumors.

Another embodiment of the present invention is a use of an anti-Epidermal Growth Factor Receptor VHH as disclosed herein, or a nucleic acid capable of encoding said polypeptide for the preparation of a medicament for treating a disorder relating to inflammatory processes and cancer.

Epidermal Growth Factor Receptor is involved in inflammatory processes, and the blocking of Epidermal Growth Factor Receptor action can have an anti-inflammatory effect, which is highly desirable in certain disease states such as, for example, inflammatory arthritis or psoriasis. Furthermore, blocking of the Epidermal Growth Factor Receptor can inhibit the growth of human tumors. Our Examples demonstrate VHHs according to the invention which bind Epidermal Growth Factor Receptor and moreover, block ligand binding to the Epidermal Growth Factor Receptor, prevent (hetero-) dimerization of the receptor and/or induce apoptosis.

The polypeptides and method of the present invention are applicable to epithelial cancers, such as lung, liver, central nervous system, bone, blood and lymphatic system, colon, breast, prostate, rectum, bladder, head and neck, ovarian, testis, pancreatic and squamos cell carcinoma. This listing of human cancers is intended to be exemplary rather than inclusive.

The method of the present invention is applicable to autoimmune diseases, such as Addison's disease (adrenal), Autoimmune diseases of the ear (ear), Autoimmune diseases of the eye (eye), Autoimmune hepatitis (liver), Autoimmune parotitis (parotid glands), Crohn's disease (intestine), Diabetes Type I (pancreas), Epididymitis (epididymis), Glomerulonephritis (kidneys), Graves' disease (thyroid). Guillain-Barre syndrome (nerve cells), Hashimoto's disease (thyroid), Hemolytic anemia (red blood cells), Systemic lupus erythematosus (multiple tissues), Male infertility (sperm), Multiple sclerosis (nerve cells), Myasthenia Gravis (neuromuscular junction), Pemphigus (primarily skin), Psoriasis (skin), Rheumatic fever (heart and joints), Rheumatoid arthritis (joint lining), Sarcoidosis (multiple tissues and organs), Scleroderma (skin and connective tissues), Sjogren's syndrome (exocrine glands, and other tissues), Spondyloarthropathies (axial skeleton, and other tissues), Thyroiditis (thyroid), Vasculitis (blood vessels). Within parenthesis is the tissue affected by the disease. This listing of autoimmune diseases is intended to be exemplary rather than inclusive.

The present invention provides a therapeutic composition comprising an anti- Epidermal Growth Factor Receptor VHH according to claim 1 which inhibits or kills human tumor cells by said VHH binding to the human Epidermal Growth Factor Receptor of said tumor cells either alone or in combination with anti-neopiastic or chemotherapeutic agents. Anti-neopiastic or chemotherapeutic agents such as doxorubicin and cisplatin are well known in the art.

Polypeptides and nucleic acids according to the present invention may be administered to a subject by conventional routes, such as intravenously. However, a special property of the anti-Epidermal Growth Factor Receptor polypeptides of the invention is that they are sufficiently small to penetrate barriers such as tissue membranes and/or tumors and act locally and act locally thereon, and they are sufficiently stable to withstand extreme environments such as in the stomach. Therefore, another aspect of the present invention relates to the delivery of anti-Epidermal Growth Factor Receptor polypeptides.

A subject according to the invention can be any mammal susceptible to treatment by therapeutic polypeptides.

Oral delivery of anti-Epidermal Growth Factor Receptor polypeptides of the invention results in the provision of such molecules in an active form at local sites that are affected by the disorder. The anti-Epidermal Growth Factor Receptor polypeptides of the invention which bind to Epidermal Growth Factor Receptor can neutralise the receptor locally, avoiding distribution throughout the whole body and thus limiting negative side-effects. Genetically modified microorganisms such as *Micrococcus lactis* are able to secrete antibody fragments. Such modified microorganisms can be used as vehicles for local production and delivery of antibody fragments in the intestine. By using a strain which produces a Epidermal Growth Factor Receptor neutralizing antibody fragment, inflammation and certain cancers could be treated.

Another aspect of the invention involves delivering anti-Epidermal Growth Factor Receptor polypeptides by using surface expression on or secretion from non-invasive bacteria, such as Gram-positive host organisms like *Lactococcus spec.* using a vector such as described in WO00/23471.

One embodiment of the present invention is an anti-Epidermal Growth Factor Receptor polypeptide as disclosed herein for use in treating, preventing and/or alleviating the symptoms of disorders susceptible to modulation by an EGFR antagonist that is able to pass through the gastric environment without the polypeptide being inactivated.

Examples of disorders are cancers and any that cause inflammation, including but not limited to rheumatoid arthritis and psoriasis. As known by persons skilled in the art, once in possession of said anti-Epidermal Growth Factor Receptor, formulation technology may be applied to release a maximum amount of polypeptide in the right location (in the stomach, in the colon, etc.). This method of delivery is important for treating, preventing and/or alleviating the symptoms of disorders whose targets are located in the gut system.

An aspect of the invention is a method for treating, preventing and/or alleviating the symptoms of a disorder susceptible to EGFR modulaters that are able to pass through the gastric environment without being inactivated, by orally administering to a subject an anti-Epidermal Growth Factor Receptor as disclosed herein.

Another embodiment of the present invention is a use of an anti-Epidermal Growth Factor Receptor polypeptide as disclosed herein for the preparation of a medicament for treating, preventing and/or alleviating the symptoms of disorders susceptible to EGFR modulators that are able to pass through the gastric environment without being inactivated.

An aspect of the invention is a method for delivering an EGFR modulator to the gut system without said compound being inactivated, by orally administering to a subject an anti-Epidermal Growth Factor Receptor polypeptide as disclosed herein.

An aspect of the invention is a method for delivering an EGFR modulator to the bloodstream of a subject without the compound being inactivated, by orally administering to a subject an anti-Epidermal Growth Factor Receptor polypeptide as disclosed herein.

Another embodiment of the present invention is an anti-Epidermal Growth Factor Receptor polypeptide as disclosed herein for use in treating, preventing and/or alleviating the symptoms or disorders susceptible to EGFR modulators delivered to the vaginal and/or rectal tract.

Examples of disorders are cancers and any that cause inflammation, including but not limited to rheumatoid arthritis and psoriasis. In a non-limiting example, a formulation according to the invention comprises an anti-Epidermal Growth Factor Receptor polypeptide as disclosed herein comprising one or more single domain antibodies directed against EGFR, in the form of a gel, cream, suppository, film, or in the form of a sponge or as a vaginal ring that slowly releases the active ingredient over time (such formulations are described in EP 707473, EP 684814, US 5629001).

An aspect of the invention is a method for treating, preventing and/or alleviating the symptoms of disorders susceptible to EGFR modulators delivered to the vaginal and/or rectal tract, by vaginally and/or rectally administering to a subject an anti-Epidermal Growth Factor Receptor polypeptide as disclosed herein.

Another embodiment of the present invention is a use of an anti-Epidermal Growth Factor Receptor polypeptide as disclosed herein for the preparation of a medicament for treating, preventing and/or alleviating the symptoms of disorders susceptible to modulation by an EGFR binding fragment delivered to the vaginal and/or rectal tract.

An aspect of the invention is a polypeptide as defined herein for use in a method for delivering an EGFR modulator to the vaginal and/or rectal tract without being said modulator being inactivated, by administering to the vaginal and/or rectal tract of a subject an anti-Epidermal Growth Factor Receptor polypeptide as disclosed herein.

An aspect of the invention is a polypeptide as defined herein for use in a method for delivering an EGFR modulator to the bloodstream of a subject without said modulator being inactivated, by administering to the vaginal and/or rectal tract of a subject an anti-Epidermal Growth Factor Receptor polypeptide as disclosed herein.

Another embodiment of the present invention is an anti-Epidermal Growth Factor Receptor polypeptide as disclosed herein, for use in treating, preventing and/or alleviating the symptoms of disorders susceptible to EGFR modulators delivered to the nose, upper respiratory tract and/or lung.

Examples of disorders are cancers and any that cause inflammation, including but not limited to inflammatory arthritis and psoriasis. In a non-limiting example, a formulation according to the invention, comprises an anti-Epidermal Growth Factor Receptor polypeptide as disclosed herein directed against EGFR in the form of a nasal spray (e.g. an aerosol) or inhaler. Since the anti-Epidermal Growth Factor Receptor polypeptide is small, it can reach its target much more effectively than therapeutic IgG molecules.

An aspect of the invention is a polypeptide as defined herein for use in a method for treating, preventing and/or alleviating the symptoms of disorders susceptible to EGFR modulators delivered to the upper respiratory tract and lung, by administering to a subject an anti-Epidermal Growth Factor Receptor polypeptide as disclosed herein, by inhalation through the mouth or nose.

Another embodiment of the present invention is a use of an anti-Epidermal Growth Factor Receptor polypeptide as disclosed herein for the preparation of a medicament for treating, preventing and/or alleviating the symptoms of disorders susceptible to modulation by an EGFR binding fragment delivered to the nose, upper respiratory tract and/or lung, without said polypeptide being Inactivated.

An aspect of the invention is a polypeptide as defined herein for use in a method for delivering an EGFR modulator to the nose, upper respiratory tract and lung without inactivation, by administering to the nose, upper respiratory tract and/or lung of a subject an anti-Epidermal Growth Factor Receptor polypeptide as disclosed herein.

An aspect of the invention is a polypeptide as defined herein for use in a method for delivering an EGFR modulator to the bloodstream of a subject without inactivation by administering to the nose, upper respiratory tract and/or lung of a subject an anti-Epidermal Growth Factor Receptor polypeptide as disclosed herein.

One embodiment of the present invention is an anti-Epidermal Growth Factor Receptor polypeptide as disclosed herein for use in treating, preventing and/or alleviating the symptoms of disorders susceptible to EGFR modulators delivered to the intestinal mucosa, wherein said disorder increases the permeability of the intestinal mucosa. Because of their small size, an anti-Epidermal Growth Factor Receptor polypeptide as disclosed herein can pass through the intestinal mucosa and reach the bloodstream more efficiently in subjects suffering from disorders which cause an increase in the permeability of the Intestinal mucosa, for example Crohn's disease.

An aspect of the invention is a polypeptide as defined herein for use in a method for treating, preventing and/or alleviating the symptoms of disorders susceptible to EGFR modulators delivered to the intestinal mucosa, wherein said disorder increases the permeability of the intestinal mucosa, by orally administering to a subject an anti-Epidermal Growth Factor Receptor polypeptide as disclosed herein.

This process can be even further enhanced by an additional aspect of the present invention - the use of active transport carriers. In this aspect of the invention, VHH is fused to a carrier that enhances the transfer through the intestinal wall into the bloodstream. In a non-limiting example, this "carrier" is a second VHH which is fused to the therapeutic VHH. Such fusion constructs are made using methods known in the art. The "carrier" VHH binds specifically to a receptor on the intestinal wall which induces an active transfer through the wall.

Another embodiment of the present invention is a use of an anti-Epidermal Growth Factor Receptor polypeptide as disclosed herein for the preparation of a medicament for treating, preventing and/or alleviating the symptoms of disorders susceptible EGFR modulators delivered to the intestinal mucosa, wherein said disorder increases the permeability of the intestinal mucosa.

An aspect of the invention is a polypeptide as defined herein for use in a method for delivering an EGFR modulator to the intestinal mucosa without being inactivated, by administering orally to a subject an anti-Epidermal Growth Factor Receptor polypeptide comprising one or more single domain antibodies directed against EGFR.

An aspect of the invention is a polypeptide as defined herein for use in a method for delivering an EGFR modulator to the bloodstream of a subject without being inactivated, by administering orally to a subject an anti-Epidermal Growth Factor Receptor polypeptide comprising one or more single domain antibodies directed against EGFR.

This process can be even further enhanced by an additional aspect of the present invention - the use of active transport carriers. In this aspect of the invention, an anti-Epidermal Growth Factor Receptor polypeptide as described herein is fused to a carrier that enhances the transfer through the Intestinal wall into the bloodstream. In a non-limiting example, this "carrier" is a VHH which is fused to said polypeptide. Such fusion constructs made using methods known in the art. The "carrier" VHH binds specifically to a receptor on the intestinal wall which induces an active transfer through the wall.

One embodiment of the present invention is an anti-Epidermal Growth Factor Receptor polypeptide as disclosed herein for use in treating, preventing and/or alleviating the symptoms of disorders susceptible to EGFR modulator that is able to pass through the tissues beneath the tongue effectively. A formulation of said an anti-Epidermal Growth Factor Receptor polypeptide as disclosed herein, for example, a tablet, spray, drop is placed under the tongue and adsorbed through the mucus membranes into the capillary network under the tongue.

An aspect of the invention is a polypeptide as defined herein for use in a method for treating, preventing and/or alleviating the symptoms of disorders susceptible to modulation by a therapeutic compound that is able to pass through the tissues beneath the tongue effectively, by sublingually administering to a subject an anti-Epidermal Growth Factor Receptor polypeptide as disclosed herein.

Another embodiment of the present invention is a use of an anti-Epidermal Growth Factor Receptor polypeptide as disclosed herein for the preparation of a medicament for treating, preventing and/or alleviating the symptoms of disorders susceptible to an EGFR modulator that is able to pass through the tissues beneath the tongue.

An aspect of the invention is a polypeptide as defined herein for use in a method for delivering an EGFR modulator to the tissues beneath the tongue without being inactivated, by administering sublingually to a subject an anti-Epidermal Growth Factor Receptor polypeptide as disclosed herein.

An aspect of the invention is a polypeptide as defined herein for use in a method for delivering an EGFR modulator to the bloodstream of a subject without being inactivated, by administering orally to a subject an anti-Epidermal Growth Factor Receptor polypeptide as disclosed herein.

One embodiment of the present invention is an anti-Epidermal Growth Factor Receptor polypeptide as disclosed herein for use in treating, preventing and/or alleviating the symptoms of disorders susceptible to an EGFR modulator that is able to pass through the skin effectively.

Examples of disorders are cancers and any that cause inflammation, including but not limited to rheumatoid arthritis and psoriasis. A formulation of said an anti-Epidermal Growth Factor Receptor polypeptide, for example, a cream, film, spray, drop, patch, is placed on the skin and passes through.

An aspect of the invention is a polypeptide as defined herein for use in a method for treating, preventing and/or alleviating the symptoms of disorders susceptible to an EGFR modulator that is able to pass through the skin effectively, by topically administering to a subject an anti-Epidermal Growth Factor Receptor polypeptide as disclosed herein.

Another embodiment of the present invention is a use of an anti-Epidermal Growth Factor Receptor polypeptides as disclosed herein for the preparation of a medicament for treating, preventing and/or alleviating the symptoms of disorders susceptible to modulation by an EGFR modulator that is able pass through the skin effectively.

An aspect of the invention is a polypeptide as defined herein for use in a method for delivering an EGFR modulator to the skin without being inactivated, by administering topically to a subject an anti-Epidermal Growth Factor Receptor polypeptide as disclosed herein.

An aspect of the invention is a polypeptide as defined herein for use in a method for delivering an EGFR modulator to the bloodstream of a subject, by administering topically to a subject an anti-Epidermal Growth Factor Receptor polypeptide as disclosed herein.

In another embodiment of the present invention, an anti-Epidermal Growth Factor Receptor polypeptide according to claim 1 further comprises a carrier single domain antibody (e.g. VHH) which acts as an active transport carrier for transport said anti-Epidermal Growth Factor Receptor polypeptide, the lung lumen to the blood.

Examples of disorders are cancers and any that cause inflammation, including but not limited to hypersecretion of lung mucus, rheumatoid arthritis, and psoriasis. The anti-Epidermal Growth Factor Receptor polypeptide further comprising a carrier binds specifically to a receptor present on the mucosal surface (bronchial epithelial cells) resulting in the active transport of the polypeptide from the lung lumen to the blood. The carrier single domain antibody may be fused to the anti-Epidermal Growth Factor Receptor polypeptide. Such fusion constructs made using methods known in the art and are describe herein. The "carrier" single domain antibody binds specifically to a receptor on the mucosal surface which induces an active transfer through the surface.

Also described is a method to determine which single domain antibodies (e.g. VHHs) are actively transported into the bloodstream upon nasal administration. Similarly, a naïve or immune VHH phage library can be administered nasally, and after different time points after administration, blood or organs can be isolated to rescue phages that have been actively transported to the bloodstream. A non-limiting example of a receptor for active transport from the lung lumen to the bloodstream is the Fc receptor N (FcRn). One aspect of the invention includes the VHH molecules identified by the method. Such VHH can then be used as a carrier VHH for the delivery of a therapeutic VHH to the corresponding target in the bloodstream upon nasal administration.

In one aspect of the invention, one can use an anti-Epidermal Growth Factor Receptor polypeptide as disclosed herein, in order to screen for agents that modulate the binding of said polypeptide to Epidermal Growth Factor Receptor. When identified in an assay that measures binding or said polypeptide displacement alone, agents will have to be subjected to functional testing to determine whether they would modulate the action of the Epidermal Growth Factor Receptor *in vivo.* Examples of screening assays are given below primarily in respect of SEQ ID NO: 3, though any anti-Epidermal Growth Factor Receptor polypeptide, as disclosed herein as disclosed herein may be appropriate.

In an example of a displacement experiment, phage or cells expressing Epidermal Growth Factor Receptor are incubated in binding buffer with, for example, a polypeptide represented by SEQ ID NO: 3 which has been labeled, in the presence or absence of increasing concentrations of a candidate modulator. To validate and calibrate the assay, control competition reactions using increasing concentrations of said polypeptide and which is unlabeled, can be performed. After incubation, cells are washed extensively, and bound, labeled polypeptide is measured as appropriate for the given label (*e.g*., scintillation counting, fluorescence, etc.). A decrease of at least 10% in the amount of labeled polypeptide bound in the presence of candidate modulator indicates displacement of binding by the candidate modulator. Candidate modulators are considered to bind specifically in this or other assays described herein if they displace 50% of labeled polypeptide (sub-saturating polypeptide dose) at a concentration of 1 µM or less.

Alternatively, binding or displacement of binding can be monitored by surface plasmon resonance (SPR). Surface plasmon resonance assays can be used as a quantitative method to measure binding between two molecules by the change in mass near an immobilized sensor caused by the binding or loss of binding of, for example, the polypeptide represented by SEQ ID NO: 3 from the aqueous phase to Epidermal Growth Factor Receptor, or fragment thereof immobilized in a membrane on the sensor. This change in mass is measured as resonance units versus time after injection or removal of the said polypeptide or candidate modulator and is measured using a Biacore Biosensor (Biacore AB). Epidermal Growth Factor Receptor, or fragment thereof can be for example immobilized on a sensor chip (for example, research grade CM5 chip; Biacore AB) in a thin film lipid membrane according to methods described by Salamon et al. (Salamon et al., 1996, Biophys J. 71: 283-294; Salamon et al., 2001, Biophys. J. 80: 1557-1567; Salamon et al., 1999, Trends Biochem. Sci. 24: 213-219, each of which is incorporated herein by reference). Sarrio *et al.* demonstrated that SPR can be used to detect ligand binding to the GPCR A(1) adenosine receptor immobilized in a lipid layer on the chip (Sarrio et al., 2000, Mol. Cell. Biol. 20: 5164-5174, incorporated herein by reference). Conditions for the binding of SEQ ID NO:3 to Epidermal Growth Factor Receptor, or fragment thereof in an SPR assay can be fine-tuned by one of skill in the art using the conditions reported by Sarrio *et al.* as a starting point.

SPR can assay for modulators of binding in at least two ways. First, a polypeptide represented by SEQ ID NO: 3, for example, can be pre-bound to immobilized Epidermal Growth Factor Receptor, or fragment thereof, followed by injection of candidate modulator at a concentration ranging from 0.1 nM to 1 µM. Displacement of the bound polypeptide can be quantitated, permitting detection of modulator binding. Alternatively, the membrane-bound Epidermal Growth Factor Receptor, or fragment thereof can be pre-incubated with a candidate modulator and challenged with, for example, a polypeptide represented by SEQ ID NO: 3. A difference in binding affinity between said polypeptide and Epidermal Growth Factor Receptor, or fragment thereof pre-incubated with the modulator, compared with that between said polypeptide and Epidermal Growth Factor Receptor, or fragment thereof in absence of the modulator will demonstrate binding or displacement of said polypeptide in the presence of modulator. In either assay, a decrease of 10% or more in the amount of said polypeptide bound in the presence of candidate modulator, relative to the amount of said polypeptide bound in the absence of candidate modulator indicates that the candidate modulator inhibits the interaction of Epidermal Growth Factor Receptor, or fragment thereof and said polypeptide.

Another method of detecting inhibition of binding of, for example, a polypeptide represented by SEQ ID NO: 3, to Epidermal Growth Factor Receptor, or fragment thereof uses fluorescence resonance energy transfer (FRET). FRET is a quantum mechanical phenomenon that occurs between a fluorescence donor (D) and a fluorescence acceptor (A) in close proximity to each other (usually < 100 A of separation) if the emission spectrum of D overlaps with the excitation spectrum of A. The molecules to be tested, *e.g*. a polypeptide represented by SEQ ID NO: 3 and an Epidermal Growth Factor Receptor, or fragment thereof, are labeled with a complementary pair of donor and acceptor fluorophores. While bound closely together by the Epidermal Growth Factor Receptor: polypeptide interaction, the fluorescence emitted upon excitation of the donor fluorophore will have a different wavelength from that emitted in response to that excitation wavelength when the said polypeptide and Epidermal Growth Factor Receptor, or fragment thereof are not bound, providing for quantification of bound versus unbound molecules by measurement of emission intensity at each wavelength. Donor fluorophores with which to label the Epidermal Growth Factor Receptor, or fragment thereof are well known in the art. Of particular interest are variants of the A. Victoria GFP known as Cyan FP (CFP, Donor (D)) and Yellow FP (YFP, Acceptor (A)). As an example, the YFP variant can be made as a fusion protein with Epidermal Growth Factor Receptor, or fragment thereof. Vectors for the expression of GFP variants as fusions (Clontech) as well as fluorophore-labeled reagents (Molecular Probes) are known in the art. The addition of a candidate modulator to the mixture of fluorescently-labeled polypeptide and YFP-Epidermal Growth Factor Receptor will result in an inhibition of energy transfer evidenced by, for example, a decrease in YFP fluorescence relative to a sample without the candidate modulator. In an assay using FRET for the detection of Epidermal Growth Factor Receptor: polypeptide interaction, a 10% or greater decrease in the intensity of fluorescent emission at the acceptor wavelength in samples containing a candidate modulator, relative to samples without the candidate modulator, indicates that the candidate modulator inhibits the Epidermal Growth Factor Receptor:polypeptide interaction.

A sample as used herein may be any biological sample containing Epidermal Growth Factor Receptor such as clinical (*e.g*. cell fractions, whole blood, plasma, serum, tissue, cells, etc.), derived from clinical, agricultural, forensic, research, or other possible samples. The clinical samples may be from human or animal origin. The sample analyzed can be both solid or liquid in nature. It is evident when solid materials are used, these are first dissolved in a suitable solution

A variation on FRET uses fluorescence quenching to monitor molecular interactions. One molecule in the interacting pair can be labeled with a fluorophore, and the other with a molecule that quenches the fluorescence of the fluorophore when brought into close apposition with it. A change in fluorescence upon excitation is indicative of a change in the association of the molecules tagged with the fluorophore:quencher pair. Generally, an increase in fluorescence of the labelled Epidermal Growth Factor Receptor, or fragment thereof is indicative that anti-Epidermal Growth Factor Receptor polypeptide bearing the quencher has been displaced. For quenching assays, a 10% or greater increase in the intensity of fluorescent emission in samples containing a candidate modulator, relative to samples without the candidate modulator, indicates that the candidate modulator inhibits Epidermal Growth Factor Receptor:anti-Epidermal Growth Factor Receptor polypeptide interaction.

In addition to the surface plasmon resonance and FRET methods, fluorescence polarization measurement is useful to quantify binding. The fluorescence polarization value for a fluorescently-tagged molecule depends on the rotational correlation time or tumbling rate. Complexes, such as those formed by Epidermal Growth Factor Receptor, or fragment thereof associating with a fluorescently labeled anti-Epidermal Growth Factor Receptor polypeptide, have higher polarization values than uncomplexed, labeled polypeptide. The inclusion of a candidate inhibitor of the Epidermal Growth Factor Receptor: anti-Epidermal Growth Factor Receptor polypeptide interaction results in a decrease in fluorescence polarization, relative to a mixture without the candidate inhibitor, if the candidate inhibitor disrupts or inhibits the interaction of Epidermal Growth Factor Receptor, or fragment thereof with said polypeptide. Fluorescence polarization is well suited for the identification of small molecules that disrupt the formation of Epidermal Growth Factor Receptor: anti-Epidermal Growth Factor Receptor polypeptide complexes. A decrease of 10% or more in fluorescence polarization in samples containing a candidate modulator, relative to fluorescence polarization in a sample lacking the candidate modulator, indicates that the candidate modulator inhibits the Epidermal Growth Factor Receptor: anti-Epidermal Growth Factor Receptor polypeptide interaction.

Another alternative for monitoring Epidermal Growth Factor Receptor:anti-Epidermal Growth Factor Receptor polypeptide interactions uses a biosensor assay. ICS biosensors have been described in the art (Australian Membrane Biotechnology Research Institute; Cornell B, Braach-Maksvytis V, King L, Osman P, Raguse B, Wieczorek L, and Pace R. "A biosensor that uses ion-channel switches" Nature 1997, 387, 580). In this technology, the association of Epidermal Growth Factor Receptor , or fragment thereof and an anti-Epidermal Growth Factor Receptor polypeptide is coupled to the closing of gramacidin-facilitated ion channels in suspended membrane bilayers and thus to a measurable change in the admittance (similar to impedence) of the biosensor. This approach is linear over six orders of magnitude of admittance change and is ideally suited for large scale, high throughput screening of small molecule combinatorial libraries. A 10% or greater change (increase or decrease) in admittance in a sample containing a candidate modulator, relative to the admittance of a sample lacking the candidate modulator, indicates that the candidate modulator inhibits the interaction of Epidermal Growth Factor Receptor, or fragment thereof and said polypeptide. It is important to note that in assays testing the interaction of Epidermal Growth Factor Receptor, or fragment thereof with an anti-Epidermal Growth Factor Receptor polypeptide, it is possible that a modulator of the interaction need not necessarily interact directly with the domain(s) of the proteins that physically interact with said polypeptide. It is also possible that a modulator will interact at a location removed from the site of interaction and cause, for example, a conformational change in the Epidermal Growth Factor Receptor. Modulators (inhibitors or agonists) that act in this manner are nonetheless of interest as agents to modulate the binding of Epidermal Growth Factor Receptor to its receptor.

Any of the binding assays described can be used to determine the presence of an agent in a sample, e.g., a tissue sample, that binds to Epidermal Growth Factor Receptor , or fragment thereof, or that affects the binding of, for example, a polypeptide represented by SEQ ID NO: 3 to the Epidermal Growth Factor Receptor , or fragment thereof. To do so an Epidermal Growth Factor Receptor, or fragment thereof is reacted with said polypeptide in the presence or absence of the sample, and polypeptide binding is measured as appropriate for the binding assay being used. A decrease of 10% or more in the binding of said polypeptide indicates that the sample contains an agent that modulates the binding of said polypeptide to the Epidermal Growth Factor Receptor , or fragment thereof.

Of course, the above-generalized methods might easily be applied to screening for candidate modulators which alter the binding between any anti-Epidermal Growth Factor Receptor polypeptide of the invention, and Epidermal Growth Factor Receptor or a fragment thereof.

A cell that is useful according to the invention is preferably selected from the group consisting of bacterial cells such as, for example, *E*. *coli,* yeast cells such as, for example, *S*. *cerevisiae, P. pastoris*, insect cells or mammalian cells.

A cell that is useful according to the invention can be any cell into which a nucleic add sequence encoding a polypeptide comprising an anti-Epidermal Growth Factor Receptor of the invention, an homologous sequence thereof, a functional portion thereof, a functional portion of an homologous sequence thereof or a mutant variant thereof according to the invention can be introduced such that the polypeptide is expressed at natural levels or above natural levels, as defined herein. Preferably a polypeptide of the invention that is expressed in a cell exhibits normal or near normal pharmacology, as defined herein. Most preferably a polypeptide of the invention that is expressed in a cell comprises the nucleotide sequence capable of encoding any one of the amino acid sequences presented in Table 5 or capable of encoding an amino acid sequence that is at least 70% identical to the amino acid sequence presented in Table 5.

According to a preferred embodiment of the present invention, a cell is selected from the group consisting of COS7-cells, a CHO cell, a LM (TK-) cell, a NIH-3T3 cell, HEK-293 cell, K-562 cell or a 1321 N1 astrocytoma cell but also other transfectable cell lines.

In general, "therapeutically effective amount", "therapeutically effective dose" and "effective amount" means the amount needed to achieve the desired result or results (modulating Epidermal Growth Factor Receptor binding; treating or preventing cancer or inflammation). One of ordinary skill in the art will recognize that the potency and, therefore, an "effective amount" can vary for the various compounds that modulate Epidermal Growth Factor Receptor binding used in the invention. One skilled in the art can readily assess the potency of the compound.

As used herein, the term "compound" refers to an anti-Epidermal Growth Factor Receptor polypeptide of the present invention, or a nucleic acid capable of encoding said polypeptide or an agent identified according to the screening method described herein or said polypeptide comprising one or more derivatized amino acids.

By "pharmaceutically acceptable" is meant a material that is not biologically or otherwise undesirable, *i.e.,* the material may be administered to an individual along with the compound without causing any undesirable biological effects or interacting in a deleterious manner with any of the other components of the pharmaceutical composition in which it is contained.

Polypeptides of a human-like class of VHH's as disclosed herein is useful for treating or preventing conditions in a subject and comprises administering a pharmaceutically effective amount of a compound or composition.

Polypeptides of the present invention are useful for treating or preventing conditions relating to canecr, rheumatoid arthritis and psoriasis in a subject and comprises administering a pharmaceutically effective amount of a compound or composition that binds Epidermal Growth Factor Receptor.

The anti-Epidermal Growth Factor Receptor polypeptides as disclosed here in are useful for treating or preventing conditions relating to cancer, rheumatoid arthritis and psoriasis in a subject and comprises administering a pharmaceutically effective amount of a compound combination with another, such as, for example, doxorubicin.

The present invention is not limited to the administration of formulations comprising a single compound of the invention. It is within the scope of the invention to provide combination treatments wherein a formulation is administered to a patient in need thereof that comprises more than one compound of the invention.

Conditions mediated by Epidermal Growth Factor Receptor include, but are not limited cancer, rheumatoid arthritis and psoriasis.

A compound useful in the present invention can be formulated as pharmaceutical compositions and administered to a mammalian host, such as a human patient or a domestic animal in a variety of forms adapted to the chosen route of administration, *i.e.,* orally or parenterally, intranassally by inhalation, intravenous, intramuscular, topical or subcutaneous routes.

A compound of the present invention can also be administered using gene therapy methods of delivery. See, *e.g*., U.S. Patent No. 5,399,346, which is incorporated by reference in its entirety. Using a gene therapy method of delivery, primary cells transfected with the gene for the compound of the present invention can additionally be transfected with tissue specific promoters to target specific organs, tissue, grafts, tumors, or cells and can additionally be transfected with signal and stabilization sequences for subcellularly localized expression.

Thus, the present compound may be systemically administered, *e.g*., orally, in combination with a pharmaceutically acceptable vehicle such as an inert diluent or an assimilable edible carrier. They may be enclosed in hard or soft shell gelatin capsules, may be compressed into tablets, or may be incorporated directly with the food of the patient's diet. For oral therapeutic administration, the active compound may be combined with one or more excipients and used in the form of ingestible tablets, buccal tablets, troches, capsules, elixirs, suspensions, syrups, wafers, and the like. Such compositions and preparations should contain at least 0.1% of active compound. The percentage of the compositions and preparations may, of course, be varied and may conveniently be between about 2 to about 60% of the weight of a given unit dosage form. The amount of active compound in such therapeutically useful compositions is such that an effective dosage level will be obtained.

The tablets, troches, pills, capsules, and the like may also contain the following: binders such as gum tragacanth, acacia, corn starch or gelatin; excipients such as dicalcium phosphate; a disintegrating agent such as corn starch, potato starch, alginic acid and the like; a lubricant such as magnesium stearate; and a sweetening agent such as sucrose, fructose, lactose or aspartame or a flavoring agent such as peppermint, oil of wintergreen, or cherry flavoring may be added. When the unit dosage form is a capsule, it may contain, in addition to materials of the above type, a liquid carrier, such as a vegetable oil or a polyethylene glycol. Various other materials may be present as coatings or to otherwise modify the physical form of the solid unit dosage form. For instance, tablets, pills, or capsules may be coated with gelatin, wax, shellac or sugar and the like. A syrup or elixir may contain the active compound, sucrose or fructose as a sweetening agent, methyl and propylparabens as preservatives, a dye and flavoring such as cherry or orange flavor. Of course, any material used in preparing any unit dosage form should be pharmaceutically acceptable and substantially non-toxic in the amounts employed. In addition, the active compound may be incorporated into sustained-release preparations and devices.

The active compound may also be administered intravenously or intraperitoneally by infusion or injection. Solutions of the active compound or its salts can be prepared in water, optionally mixed with a nontoxic surfactant. Dispersions can also be prepared in glycerol, liquid polyethylene glycols, triacetin, and mixtures thereof and in oils. Under ordinary conditions of storage and use, these preparations contain a preservative to prevent the growth of microorganisms.

The pharmaceutical dosage forms suitable for injection or infusion can include sterile aqueous solutions or dispersions or sterile powders comprising the active ingredient which are adapted for the extemporaneous preparation of sterile injectable or infusible solutions or dispersions, optionally encapsulated in liposomes. In all cases, the ultimate dosage form must be sterile, fluid and stable under the conditions of manufacture and storage. The liquid carrier or vehicle can be a solvent or liquid dispersion medium comprising, for example, water, ethanol, a polyol (for example, glycerol, propylene glycol, liquid polyethylene glycols, and the like), vegetable oils, nontoxic glyceryl esters, and suitable mixtures thereof. The proper fluidity can be maintained, for example, by the formation of liposomes, by the maintenance of the required particle size in the case of dispersions or by the use of surfactants. The prevention of the action of microorganisms can be brought about by various antibacterial and antifungal agents, for example, parabens, chlorobutanol, phenol, sorbic acid, thimerosal, and the like. In many cases, it will be preferable to include isotonic agents, for example, sugars, buffers or sodium chloride. Prolonged absorption of the injectable compositions can be brought about by the use in the compositions of agents delaying absorption, for example, aluminum monostearate and gelatin.

Sterile injectable solutions are prepared by incorporating the active compound in the required amount in the appropriate solvent with various of the other ingredients enumerated above, as required, followed by filter sterilization. In the case of sterile powders for the preparation of sterile injectable solutions, the preferred methods of preparation are vacuum drying and the freeze drying techniques, which yield a powder of the active ingredient plus any additional desired ingredient present in the previously sterile-filtered solutions.

For topical administration, the present compound may be applied in pure form, i.e., when they are liquids. However, it will generally be desirable to administer them to the skin as compositions or formulations, in combination with a dermatologically acceptable carrier, which may be a solid or a liquid.

Useful solid carriers include finely divided solids such as talc, clay, microcrystalline cellulose, silica, alumina and the like. Useful liquid carriers include water, hydroxyalkyls or glycols or water-alcohol/glycol blends, in which the present compound can be dissolved or dispersed at effective levels, optionally with the aid of non-toxic surfactants. Adjuvants such as fragrances and additional antimicrobial agents can be added to optimize the properties for a given use. The resultant liquid compositions can be applied from absorbent pads, used to impregnate bandages and other dressings, or sprayed onto the affected area using pump-type or aerosol sprayers.

Thickeners such as synthetic polymers, fatty acids, fatty acid salts and esters, fatty alcohols, modified celluloses or modified mineral materials can also be employed with liquid carriers to form spreadable pastes, gels, ointments, soaps, and the like, for application directly to the skin of the user.

Examples of useful dermatological compositions which can be used to deliver the compound to the skin are known to the art; for example, see Jacquet et al. (U.S. Pat. No. 4,608,392), Geria (U.S. Pat. No. 4,992,478), Smith et al. (U.S. Pat. No. 4,559,157) and Wortzman (U.S. Pat. No. 4,820,508).

Useful dosages of the compound can be determined by comparing their *in vitro* activity, and *in vivo* activity in animal models. Methods for the extrapolation of effective dosages in mice, and other animals, to humans are known to the art; for example, see U.S. Pat. No. 4,938,949.

Generally, the concentration of the compound(s) in a liquid composition, such as a lotion, will be from about 0.1-25 wt-%, preferably from about 0.5-10 wt-%. The concentration in a semi-solid or solid composition such as a gel or a powder will be about 0.1-5 wt-%, preferably about 0.5-2.5 wt-%.

The amount of the compound, or an active salt or derivative thereof, required for use in treatment will vary not only with the particular salt selected but also with the route of administration, the nature of the condition being treated and the age and condition of the patient and will be ultimately at the discretion of the attendant physician or clinician. Also the dosage of the compound varies depending on the target cell, tumor, tissue, graft, or organ.

The desired dose may conveniently be presented in a single dose or as divided doses administered at appropriate intervals, for example, as two, three, four or more sub-doses per day. The sub-dose itself may be further divided, *e.g*., into a number of discrete loosely spaced administrations; such as multiple inhalations from an insufflator or by application of a plurality of drops into the eye.

An administration regimen could include long-term, daily treatment. By "long-term" is meant at least two weeks and preferably, several weeks, months, or years of duration. Necessary modifications in this dosage range may be determined by one of ordinary skill in the art using only routine experimentation given the teachings herein. See Remington's Pharmaceutical Sciences (Martin, E.W., ed. 4), Mack Publishing Co., Easton, PA. The dosage can also be adjusted by the individual physician in the event of any complication.

### Candidate modulators

Also described is an agent that is a modulator of interactions between Epidermal Growth Factor Receptor and its ligand.

The candidate agent may be a synthetic agent, or a mixture of agents, or may be a natural product (*e.g*. a plant extract or culture supematant). A candidate agent includes a small molecule that can be synthesized, a natural extract, peptides, proteins, carbohydrates, lipids etc.

Candidate modulator agents from large libraries of synthetic or natural agents can be screened. Numerous means are currently used for random and directed synthesis of saccharide, peptide, and nucleic add based agents. Synthetic agent libraries are commercially available from a number of companies including Maybridge Chemical Co. (Trevillet, Cornwall, UK), Comgenex (Princeton, NJ), Brandon Associates (Merrimack, NH), and Microsource (New Milford, CT). A rare chemical library is available from Aldrich (Milwaukee, WI). Combinatorial libraries are available and can be prepared. Alternatively, libraries of natural agents in the form of bacterial, fungal, plant and animal extracts are available from e.g., Pan Laboratories (Bothell, WA) or MycoSearch (NC), or are readily producible by methods well known in the art. Additionally, natural and synthetically produced libraries and agents are readily modified through conventional chemical, physical, and biochemical means.

Useful agents may be found within numerous chemical classes. Useful agents may be organic agents, or small organic agents. Small organic agents have a molecular weight of more than 50 yet less than about 2,500 daltons, preferably less than about 750, more preferably less than about 350 daltons. Exemplary classes include heterocycles, peptides, saccharides, steroids, and the like. The agents may be modified to enhance efficacy, stability, pharmaceutical compatibility, and the like. Structural identification of an agent may be used to identify, generate, or screen additional agents. For example, where peptide agents are identified, they may be modified in a variety of ways to enhance their stability, such as using an unnatural amino acid, such as a D-amino acid, particularly D-alanine, by functionalizing the amino or carbolic terminus, e.g. for the amino group, acylation or alkylation, and for the carboxyl group, esterification or amidification, or the like.

For primary screening, a useful concentration of a candidate agent according to the invention is from about 10 mM to about 100 µM or more (*i.e.* 1 mM, 10 mM, 100 mM, 1 M etc.). The primary screening concentration will be used as an upper limit, along with nine additional concentrations, wherein the additional concentrations are determined by reducing the primary screening concentration at half-log intervals (*e.g*. for 9 more concentrations) for secondary screens or for generating concentration curves.

### High throughput screening kit

A high throughput screening kit as described herein comprises all the necessary means and media for performing the detection of an agent that modulates Epidermal Growth Factor Receptor /ligand interactions by interacting with Epidermal Growth Factor Receptor, or fragment thereof in the presence of a polypeptide, preferably at a concentration in the range of 1µM to 1 mM.

The kit comprises the following. Recombinant cells comprising and expressing the nucleotide sequence encoding Epidermal Growth Factor Receptor , or fragment thereof, which are grown according to the kit on a solid support, such as a microtiter plate, more preferably a 96 well microtiter plate, according to methods well known to the person skilled in the art especially as described in WO 00/02045. Alternatively Epidermal Growth Factor Receptor, or fragment thereof is supplied in a purified form to be immobilized on, for example, a 96 well microtiter plate by the person skilled in the art. Alternatively Epidermal Growth Factor Receptor , or fragment thereof is supplied in the kit pre-immobilized on, for example, a 96 well microtiter plate. The Epidermal Growth Factor Receptor may be whole Epidermal Growth Factor Receptor or a fragment thereof.

Modulator agents, at concentrations from about 1 µM to 1 mM or more, are added to defined wells in the presence of an appropriate concentration of anti-Epidermal Growth Factor Receptor polypeptide according to claim 1, an homologous sequence thereof, a functional portion thereof or a functional portion of an homologous sequence thereof, said concentration of said polypeptide preferably in the range of 1 µM to 1 mM. Kits may contain one or more anti-Epidermal Growth Factor Receptor polypeptide (*e.g*. one or more of a polypeptide represented by any of the SEQ ID NOs: 1 to 15 or other anti-Epidermal Growth Factor Receptor polypeptides, an homologous sequence thereof, a functional portion thereof or a functional portion of an homologous sequence thereof).

Binding assays are performed as according to the methods already disclosed herein and the results are compared to the baseline level of, for example Epidermal Growth Factor Receptor, or fragment thereof binding to an anti-Epidermal Growth Factor Receptor polypeptide, an homologous sequence thereof, a functional portion thereof or a functional portion of an homologous sequence thereof , but in the absence of added modulator agent. Wells showing at least 2 fold, preferably 5 fold, more preferably 10 fold and most preferably a 100 fold or more increase or decrease in Epidermal Growth Factor Receptor - polypeptide binding (for example) as compared to the level of activity in the absence of modulator, are selected for further analysis.

### Other Useful Kits

Also described are kits useful for screening for modulators of Epidermal Growth Factor Receptor /ligand binding, as well as kits useful for diagnosis of disorders characterized by dysfunction of Epidermal Growth Factor Receptor signaling. Also described are kits useful for screening for modulators of disorders as well as kits for their diagnosis, said disorders characterized by one or more process involving Epidermal Growth Factor Receptor. Useful kits can include an isolated Epidermal Growth Factor Receptor, or fragment thereof. Alternatively, or in addition, a kit can comprise cells transformed to express Epidermal Growth Factor Receptor, or fragment thereof. A kit can comprise a polynucleotide encoding Epidermal Growth Factor Receptor, or fragment thereof. A kit may comprise the specific primers useful for amplification of Epidermal Growth Factor Receptor, or fragment thereof. Useful kits can comprise an isolated Epidermal Growth Factor Receptor polypeptide, a homologue thereof, or a functional portion thereof. A kit can comprise cells transformed to express said polypeptide. Kits may contain more than one polypeptide. A kit can comprise a polynucleotide encoding Epidermal Growth Factor Receptor, or fragment thereof. A kit may comprise the specific primers useful for amplification of a macromolecule such as, for example, Epidermal Growth Factor Receptor, or a fragment thereof. All kits will comprise the stated items or combinations of items and packaging materials therefore. Kits will also include instructions for use.

### EXAMPLES

The invention is illustrated by the following non-limiting example.

### Example 1: Immunization

After approval of the Ethical Committee of the Faculty of Veterinary Medicine (University Ghent, Belgium), 4 llamas (024, 025, 026 and 027) were immunized with the tumor antigen epidermal growth factor receptor (EGFR) according to all current animal welfare regulations. To generate an antibody dependent immune response (table 1), two animals were injected with intact human vulvar squamous carcinoma cells (A431, ATCC CRL 1555), expressing EGFR on its cell surface, while A431 derived membrane extracts were administered to two other llamas (026 and 027). Each animal received seven doses of subcutaneously administered antigens at weekly intervals (table 1). When immunizing with intact cells, each dose consisted of 10⁸ freshly harvested A431 cells. The dose for immunization with membrane extracts consisted of vesicles prepared from 10⁸ A431 cells. Vesicles were prepared according to Cohen and colleagues (Cohen S, Ushiro H, Stoscheck C, Chinkers M, 1982. A native 170,000 epidermal growth factor receptor kinase complex from shed plasma membrane vesicles. J. Biol. Chem. 257:1523-31). Vesicles were stored at -80°C before administration. Two extra injections of eight microgram purified EGFR (Sigma) in an emulsion with the adjuvant Stimune (CEDI Diagnostics B.V., Lelystad, The Netherlands) were administered intramuscularly to llama 025 (table 1).

### Example 2: Evaluation of immune response

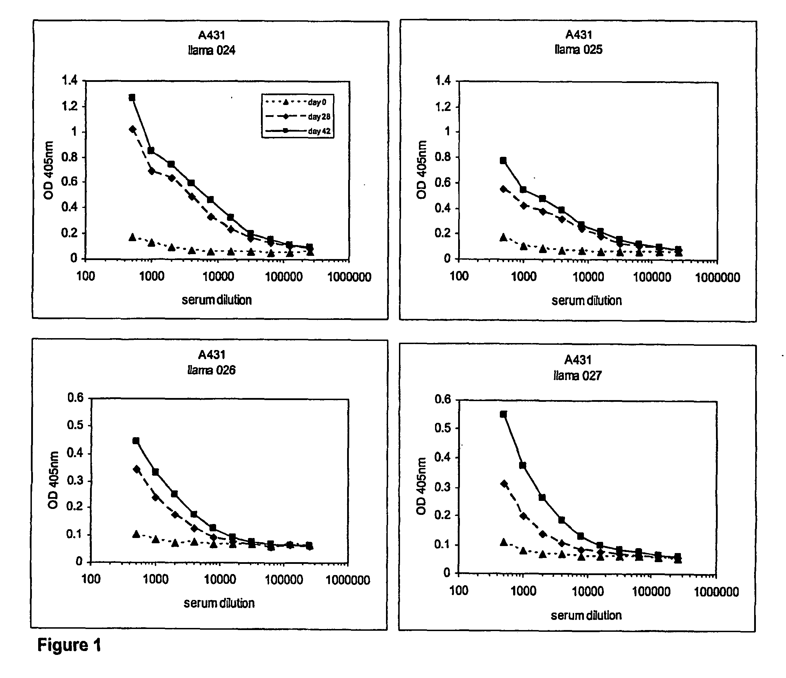

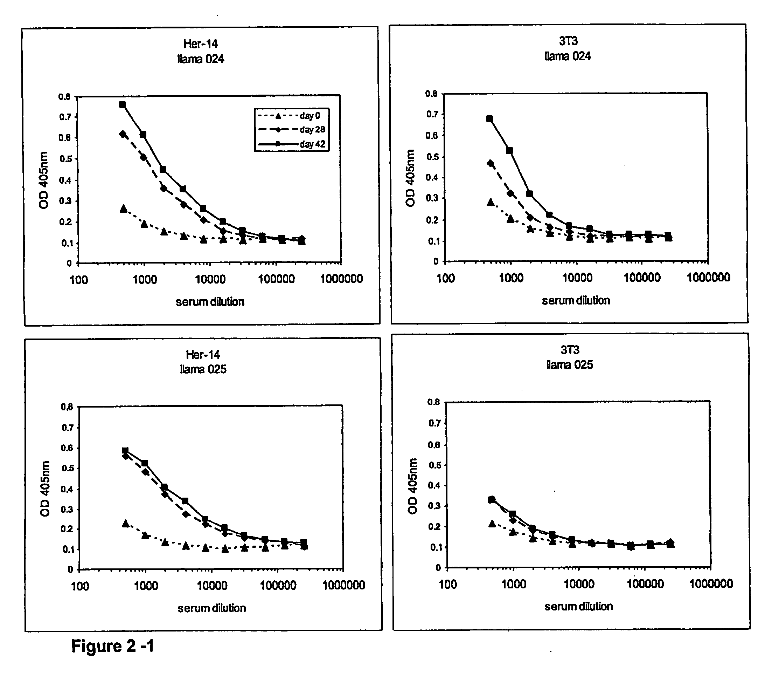

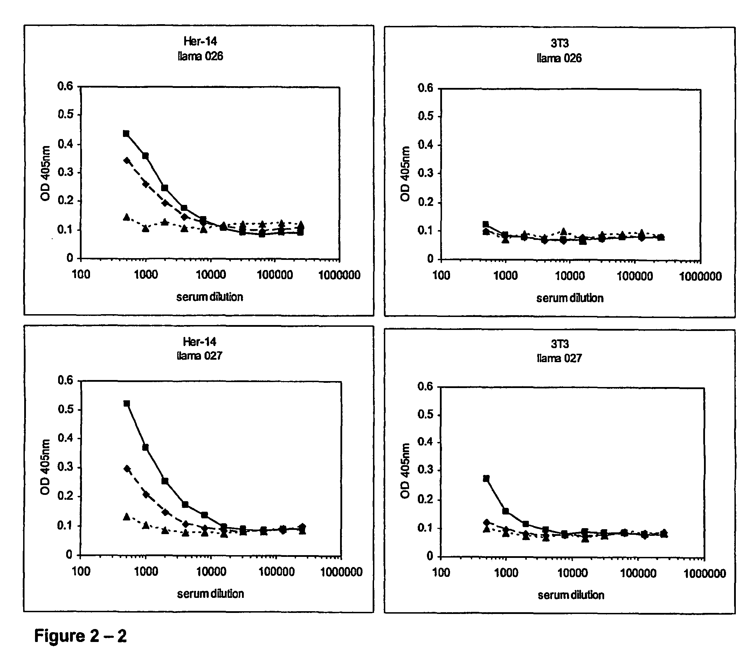

At day 0, 28 and 42, 10 ml of (pre-)immune blood was collected and serum was used to evaluate the induction of the immune responses in the 4 animals. A first ELISA was performed to verify whether the animals generated antibodies that recognized A431 epitopes. After coating a tissue-culture treated 96-well plate with gelatin (0.5% in PBS for 10 minutes), the excess of gelatin was removed and A431 cells were grown overnight in the microwells to confluency. Cells were fixed with 4% paraformaldehyde in PBS for 30 minutes at room temperature. Subsequently, the fixative was blocked with 100mM glycine in PBS for 10 minutes, followed by blocking of the wells with a 4% skim milk-PBS solution, again for 10 minutes. Serum dilutions of immunized animals were applied and A431 specific antibodies were detected with a polyclonal anti-llama antiserum developed in rabbit, followed by a secondary goat anti-rabbit horse radish peroxidase (HRP) conjugate (Dako, Denmark). For all four animals, immunization with intact cells or membrane vesicles resulted in the induction of a significant A431-specific antibody titer (figure 1). To verify whether the induced llama antibodies were EGFR specific, antibody titers in serum was evaluated on mouse fibroblasts expressing human EGFR (Her-14) and compared to the parental mouse fibroblasts cell line NIH3T3 clone 2.2 (3T3), similarly performed as described above (figure 2). Again, the serum titer of antibodies binding to Her-14 was higher compared to the titer for the parental 3T3 cells, indicating that circulating serum antibodies were EGFR specific.

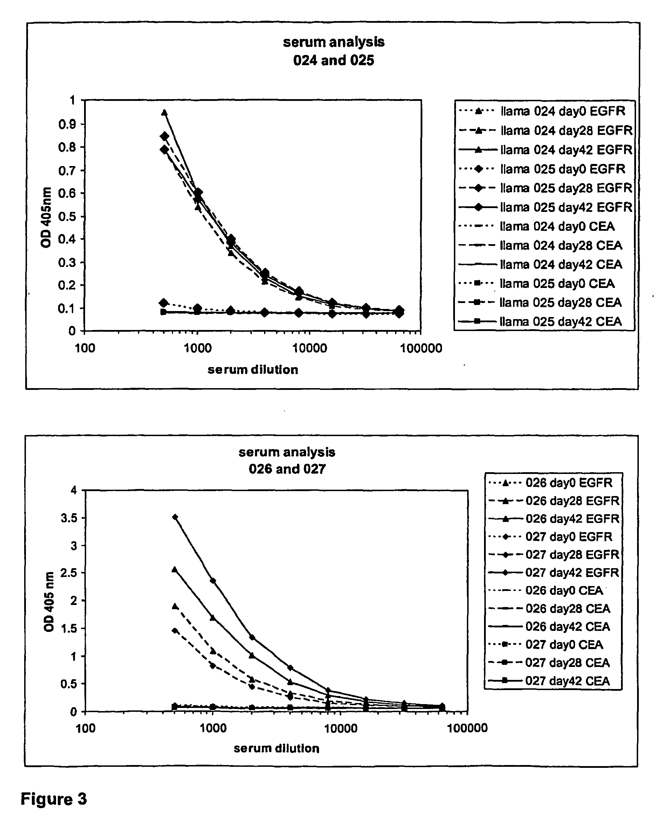

Finally, the serum response in immunized animals was verified on solid-phase coated purified EGFR. Purified EGFR (Sigma) and the irrelevant carcino embryonic antigen (CEA, Scripps), both at 1 µg/ml, were immobilized overnight at 4°C in a 96 well Maxisorp plate (Nunc). Wells were blocked with a casein solution (1% in PBS). After addition of serum dilutions, specifically bound immunoglobulins were detected using a rabbit anti-llama antiserum followed by a goat anti-rabbit alkaline phosphatase conjugate (Sigma), showing that for all animals a significant antibody dependent immune response against EGFR was induced (figure 3).

### Example 3: Cloning of the heavy-chain antibody fragment (VHH) repertoire

Since little is known on the immunoglobulin ontogeny of camelids, B-cell containing tissues of distinct origin and of different time points were collected for each animal (table 1). After tissue collection, total RNA was isolated according to the procedure described by Chomczynski and Sacchi. (Chomczynski P and Sacchi N. 1987. Single-step method of RNA isolation by acid guanidinium thiocyanate-phenol-chloroform extraction. Anal Biochem 162:156-159). The procedure to clone the VHH repertoire is based on a method described in patent application WO 03/054016. cDNA was prepared on total RNA with MMLV Reverse Transcriptase (Invitrogen) using oligo d(T) oligonucleotides (de Haard HJ, van Neer N, Reurs A, Hufton SE, Roovers RC, Henderikx P, de Bruine AP, Arends JW, Hoogenboom HR. 1999. A large non-immunized human Fab fragment phage library that permits rapid isolation and kinetic analysis of high affinity antibodies. J. Biol. Chem. 274:18218-30). The amounts of RNA of the distinct tissues used for cDNA synthesis is listed in table 2. The cDNA was purified with a phenol/chloroform extraction, followed by an ethanol precipitation and subsequently used as template to amplify the VHH repertoire. In a first PCR, the repertoire of both conventional (1.6 kb) and heavy chain (1.3 kb) antibody gene segments were amplified using a leader specific primer (ABL002) and ABL010, an oligo d(T) primer (for a list of primers see table 6). The resulting DNA fragments were separated by agarose gel electrophoresis. The amplified 1.3 kb fragment, encoding heavy-chain antibody segments was purified from the agarose gel and used as template in a nested PCR using a mixture of FR1 primers (ABL037-ABL043) and ABL010. The PCR products were digested with S*fi*I (introduced in the FR1 primer) and *BstE*II (naturally occurring in FR4). Following gel electrophoresis, the DNA fragment of approximately 400 basepairs was purified from gel and 330 ng of amplified VHH repertoire was ligated into the corresponding restriction sites of one microgram of phagemid pAX004 to obtain a library after electroporation of *Escherichia coli* TG1. pAX004 allows the production of phage particles, expressing the individual VHHs as a fusion protein with the geneIII product. The size of the libraries obtained from the distinct tissues collected from the immunized llamas is described in table 2. As a quality control, a colony PCR using the M13 reverse and a geneIII primer was performed on 24 randomly picked colonies of each library and the percentage of clones containing an insert of the correct size was calculated (table 2).

### Example 4: evaluation of the cloned repertoire

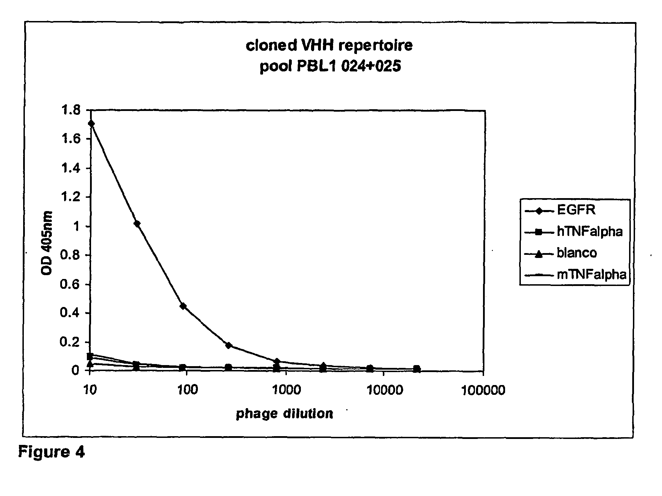

In a polyclonal phage ELISA, the specificity of the cloned phage repertoire was evaluated on EGFR and on an irrelevant antigen (TNFα). To generate recombinant virions expressing the VHH repertoire as fusion proteins with the geneIII product, the library was grown at 37°C in 10 ml 2xTY medium containing 2% glucose, and 100 µg/ml ampicillin, until the OD₆₀₀ₙₘ reached 0.5. M13KO7 phages (10¹²) were added and the mixture was incubated at 37°C for 2 x 30 minutes, first without shaking, then with shaking at 100 rpm. Cells were centrifuged for 5 minutes at 4,500 rpm at room temperature. The bacterial pellet was resuspended in 50 ml of 2xTY medium containing 100 µg/ml ampicillin and 25 µg/ml kanamycin, and incubated overnight at 37°C with vigorously shaking at 250 rpm. The overnight cultures were centrifuged for 15 minutes at 4,500 rpm at 4°C and supernatant was used to concentrate the phages. Phages were PEG precipitated (20% poly-ethylene-glycol and 1.5 M NaCl) for 30 minutes on ice and centrifuged for 20 minutes at 4, 500 rpm. The pellet was resuspended in 1 ml PBS. Phages were again PEG precipitated for 10 minutes on ice and centrifuged for 10 minutes at 14,000 rpm and 4°C. The pellet was dissolved in 1 ml PBS. One µg/ml of EGFR or TNFα was immobilized in a 96 well Maxisorp plate (Nunc) and incubated overnight at 4°C. Plates were washed 5 times with PBS/0.05%Tween-20 and wells were blocked with a casein solution (1% in PBS) and phage dilutions were added for 2 hrs at room temperature. Bound phages were detected using the anti-M13 gpVIII-HRP conjugated monoclonal antibody (Amersham Biosciences) and ABTS/H2O2 as substrate. Plates were read at 405nm after 15 minutes incubation at room temperature. An example of a phage response from a pool of phages rescued from PBL1 libraries of animals 024 and 025 is depicted in figure 4.

### Example 5: Multiple selection strategies to identify EGFR specific nanobodies

Libraries were rescued by growing the bacteria to logarithmic phase (OD₆₀₀= 0.5), followed by infection with helper phage to obtain recombinant phages expressing the repertoire of cloned VHHs on tip of the phage as gpIII fusion protein (as described in example 4). When selecting for EGFR specific antibodies, two distinct selection strategies have been followed.

### Selection by epitope specific elution

A first selection strategy was based on the fact that EGFR can be purified by affinity chromatography through ligand elution. Four different elution conditions, applying an excess of molecules that compete for the ligand binding site or overlapping epitope(s) were carried out (table 3). When selection was performed on A431 or Her-14 cells, unselected recombinant phages were mixed for 20 minutes at 4°C with 6 x 10⁸ blood cells (mainly monocytes, T- and B-cells) or 2 x 10⁷ 3T3s, respectively, to deplete for recombinant phages that recognize common, non EGFR-specific epitopes. Unbound phages were then incubated with EGFR⁺ selection cells for 2 hours followed by 6 washes with ice-cold PBS. Phages were subsequently eluted with an excess of EGF ligand, mouse monoclonal 2e9 (Defize LH, Moolenaar WH, van der Saag PT, de Laat SW 1986. Dissociation of cellular responses to epidermal growth factor using anti-receptor monoclonal antibodies. EMBO J. 5:1187-92) or EGFR antagonistic antibodies 225 and 528 (Sato JD, Kawamoto T, Le AD, Mendelsohn J, Polikoff J, Sato GH 1983. Biological effects in vitro of monoclonal antibodies to human epidermal growth factor receptors. Mol. Biol. Med. 1:511-529). All selection steps were performed at 4°C to avoid receptor mediated phage internalization. Logarithmically grown *E*. *coli* TG1 was infected with the eluted phages and grown overnight at 37°C on selective medium 2xTY Ap100 and 2% glucose. Cells were scraped and used in a next round of panning whenever required. Two or three rounds of panning were performed to enrich for EGFR specific recombinant phages (table 3). Whenever purified antigen was used for selection (table 3), EGFR was immobilized at 1 µglml on Maxisorp microtiter plates.

### Selection for internalizing VHH fragments

A second selection strategy was based on the observation that after binding of the ligand to the receptor, EGFR mediated cell signaling can be downregulated by the mechanism of receptor internalization. To identify recombinant phages that are able to internalize through cell surface molecules, the protocol described by Poul and colleagues (Poul MA, Becerril B, Nielsen UB, Morisson P, Marks JD. 2000. Selection of tumor-specific internalizing human antibodies from phage libraries. J. Mol. Biol. 301:1149-61.) was followed. Unselected recombinant phages were added to approximately 2 x 10⁷ mouse fibroblast 3T3s for 30 minutes at 4°C in ice cold binding medium (bicarbonate buffered DMEM; 10% Foetal Calf Serum; 25mM Hepes), supplemented with 2% skim milk to deplete for non-specific VHHs. Unbound phages were subsequently incubated with precooled EGFR⁺ selection cells (Her-14 or A431) in binding medium for 1.5 hours at 4°C, followed by six washes with ice-cold PBS to remove non-bound phages. Cells were covered with pre-warmed binding medium and immediately transferred to 37°C for 20 minutes, to allow internalization. Subsequently, cells were cooled down to 4°C and were stripped with mild acid (500mM Nacl; 100mM glycine pH2.5) incubations during 10 minutes to remove surface bound recombinant phages. Cells were released from extracellular matrix by trypsinization. Resuspended cells were then lyzed during 4 minutes with 100mM TEA at 4°C to release internalized phages. Logarithmically grown *E*. *coli* TG1 was infected with the eluted phages and grown overnight at 37°C on selective medium (2xTY Ap100 with 2% glucose). The libraries used for a single round of selection on A431 and in parallel on Her-14 are summarized in table 4.

### Example 6: characterization of EGFR specific nanobodies

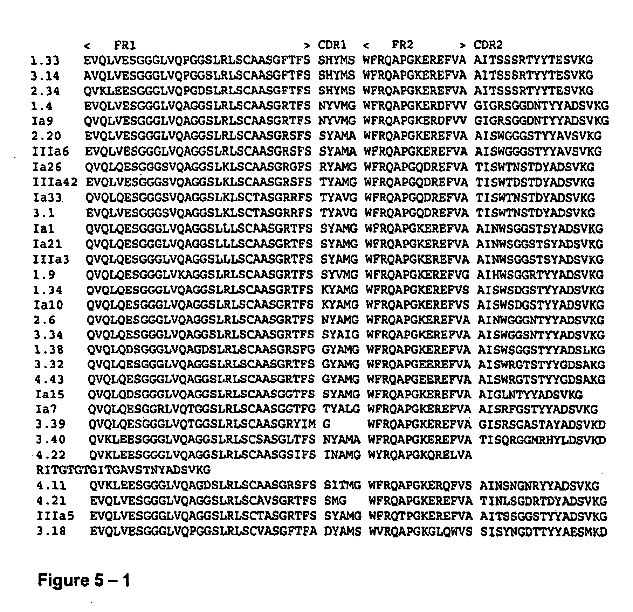

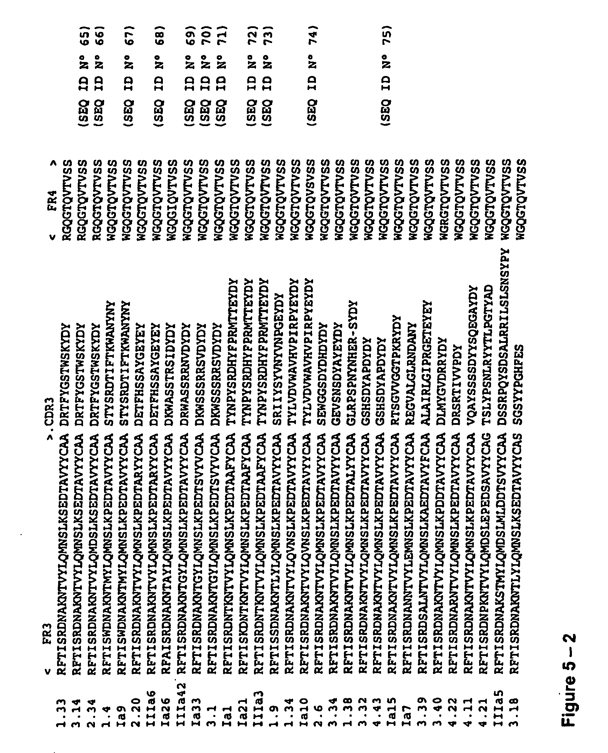

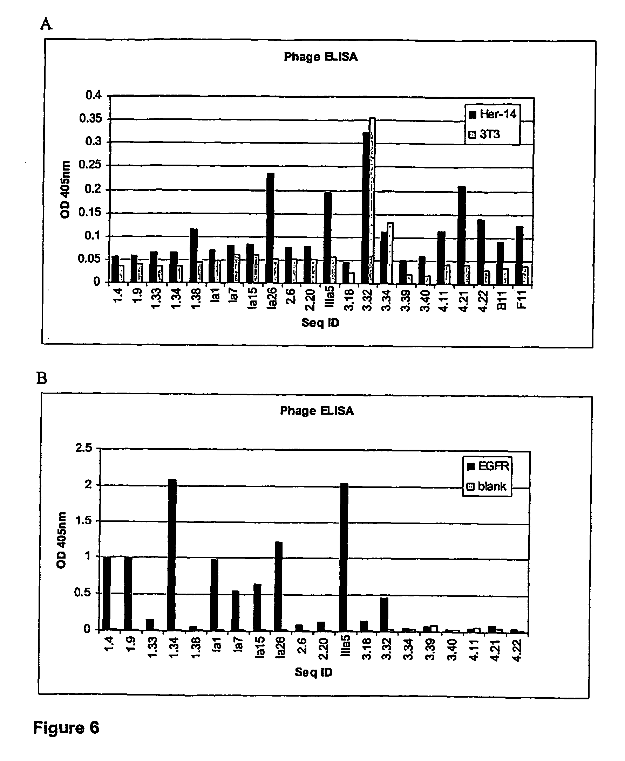

To verify EGFR specificity of individual clones after the epitope specific elution procedure of panning, a phage ELISA was performed on individual clones. 47 randomly picked clones for each selection procedure (1, 2, 3, 4, la and IIIa; table 3) were grown to logarithmic phase (OD₆₀₀= 0.5), followed by infection with helper phage to obtain recombinant phages as described in example 4. A phage ELISA was performed both on solid-phase coated EGFR (comparing to non-coated well) as on gelatin coated Her-14 cells (comparing to 3T3). The presence of EGFR specific VHH was verified by using approximately 10⁹ recombinant phage particles of each clone before detection with an anti-M13 gpVIII-HRP conjugated monoclonal antibody. With clones that scored positive in phage ELISA on cells and/or on solid-phase immobilized EGFR (table 3), a *Hin*fI fingerprint analysis was performed (data not shown). The nucleotide sequence was determined for a representative clone of each distinct fingerprint, resulting in 5, 8, 3, 4, 7, and 4 different sequences for conditions, 1, la, 2, IIIa, 3 and 4, respectively. Amino acid sequence alignment of these 31 binders (figure 5) indicated that 20 of them were unique (listed in table 5). The EGFR specificity of the 20 unique clones in phage ELISA (both on cells and on solid-phase coated EGFR) is shown in figure 6.

For the selection according to the internalization protocol, a phage ELISA on cells with a total of 84 individual clones was performed, similarly as for the clones identified by the epitope specific elution selection procedure. After *Hinf*I fingerprint analysis, nucleotide sequence determination and amino acid sequence alignment to the above described panel of 20 unique binders (data not shown), 2 new anti-EGFR clones, EGFR-B11 and clone EGFR-F11, were identified (table 5). The EGFR specificity of both clones in phage ELISA on cells is shown in figure 6, panel A.

### Example 7: EGF receptor mediated internalization of nanobodies

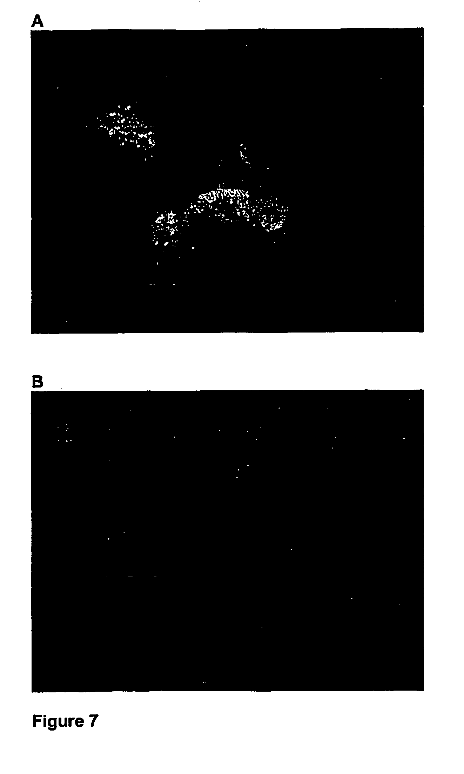

Her-14 and 3T3 cells were grown overnight on glass cover slips, washed with binding medium (see example 5) and cooled down to 4 °C for 20 minutes. Phages were prepared of nanobody EGFRIIIa42 as described in example 4 and approximately 10¹² recombinant virions, diluted in binding medium supplemented with 2% skim milk, were added to the ice cold cells for 1 hour at 4 °C. Cells were washed once with ice cold PBS to remove non bound phages. Subsequently, the cells were shifted to 37°C for 20 minutes to allow phage internalization and again cooled down to 4°C. Cells were washed twice with PBS. Following, cell surface bound phages were removed by two acid washes with stripping buffer (150 mM NaCl, 125 mM HAc) for seven minutes at room temperature. After two washes with PBS, cells were fixed with 4% paraformaldehyde in PBS for 30 minutes at room temperature, and again washed twice with PBS. Fixed cells were then permeabilized in 0.2% Triton X-100 in PBS for 5 minutes at room temperature, followed by two washes with PBS and remaining fixative was blocked with 100mM glycin in PBS for 10 minutes at room temperature. Cells were washed with PBS-0.5% (w/v) gelatin and internalized phage was visualized by staining with anti-M13 gpVIII-FITC (Amersham Biosciences) followed by an anti-mouse FITC labeled monoclonal antibody and subsequent visualization by fluorescence microscopy. Figure 7 shows that EGFRIIIa42 is able to internalize Her-14 (panel A) but not 3T3 cells (panel B).

### TABLES

**Table 1**

| **Day** | **Llama 024** | **Llama 025** | **Llama 026** | **Llama 027** |
|---|---|---|---|---|
| 0 | intact cells | intact cells | vesicles | vesicles |
| 7 | intact cells | intact cells | vesicles | vesicles |
| 14 | intact cells | intact cells | vesicles | vesicles |
| 21 | intact cells | intact cells | vesicles | vesicles |
| 28 | intact cells | intact cells | vesicles | vesicles |
| 35 | intact cells | intact cells | vesicles | vesicles |
| 42 | intact cells | intact cells | vesicles | vesicles |
| 46 | 150 ml blood sample (PBL1) | 150 ml blood sample (PBL1) | 150 ml blood sample (PBL1) lymph node | 150 ml blood sample (PBL1) lymph node |
| 47 | lymph node spleen bone marrow | | | |
| 49 | | purified EGFR | 150 ml blood sample (PBL2) | 150 ml blood sample (PBL2) |
| 55 | | purified EGFR | | |
| 59 | | 150 ml blood sample (PBL2) | | |
| 60 | | lymph node spleen bone marrow | | |

**Table 2**

| **Animal** | **Tissue** | **RNA (µg)** | **Size (x 10⁸)** | **% Insert** |
|---|---|---|---|---|
| Llama 024 | PBL1 | 200 | 0.25 | 83 |
| Llama 024 | Lymph node ileum | 40 | 2.3 | 78 |
| Llama 024 | Lymph node bow | 150 | 0.17 | 100 |
| Llama 024 | Bone marrow | 97 | 1.5 | 83 |
| Llama 024 | Spleen | 160 | 0.16 | 95 |
| Llama 025 | PBL1 | 200 | 0.06 | 95 |
| Llama 025 | Lymph node (ileum + bow) | 200 | 0.8 | 96 |
| Llama 025 | Bone marrow | 200 | 0.045 | 88 |
| Llama 025 | Spleen | 200 | 2 | 86 |
| Llama 025 | PBL2 | 200 | 0.13 | 83 |
| Llama 026 | PBL1 + lymph node | 100 + 200 | 2.46 | 85 |
| Llama 027 | PBL1 + lymph node | 100 + 200 | 1.08 | 92 |

**Table 3**

| Elution condition | Elution molecule | Selection: antigen format | | | ΦELISA Her-14 | ΦELISA EGFR | Binder families |
|---|---|---|---|---|---|---|---|
| | | Round I | Round II | Round III | | | |
| 1 | EGF | A431 | Her-14 | - | 1/47 | 24/47 | 6 |
| Ia | | | EGFR | | 5/47 | 23/47 | 8 |
| 2 | 2e9 | A431 | Her-14 | - | 2/47 | 32/47 | 5 |
| IIIa | | | EGFR | | 11/47 | 32/47 | 4 |
| 3 | 225 | A431 | A431 | Her-14 | 8/47 | 28/47 | 5 |
| | | | | EGFR | 20/47 | 31/47 | |
| 4 | 528 | A431 | A431 | Her-14 | 16/47 | 10/47 | 5 |
| | | | | EGFR | 22/47 | 29/47 | |

**Table 4**

| Library | Selection cells | Selected antibody fragment |
|---|---|---|
| Pool lymph node, bone marrow, spleen and PBL1 (024+025) | Her-14 | A2 |
| Pool bone marrow (024+025) | A431 | A4, A9, B11 |
| Pool PBL1 (024+025) | A431 | F11 |

**Table 5**

| **Seq ID** | **Name** | **Sequence** |
|---|---|---|
| 1 | EGFR-1.4 | EVQLVESGGGLVQAGGSLRLSCAASGRTFSNYVMGWFRQAPGKERDFVV GIGRSGGDNTYYADSVKGRFTISWDNAKNTMYLQMNSLKPEDTAVYYCA ASTYSRDTIFTKWANYNYWGQGTQVTVSS |
| 2 | EGFR-1.9 | QVQLQESGGGLVKAGGSLRLSCAASGRTFSSYVMGWFRQAPGKEREFVG AIHWSGGRTYYADSVKGRFTISSDNAKNTLYLQMNSLKPEDTAVYYCAA SRIIYSYVNYVNPGEYDYWGQGTQVTVSS |
| 3 | EGFR-1.33 | EVQLVESGGGLVQPGGSLRLSCAASGFTFSSHYMSWFRQAPGKEREFVA AITSSSRTYYTESVKGRFTISRDNAKNTVYLQMNSLKSEDTAVYYCAAD RTFYGSTWSKYDYRGQGTQVTVSS |
| 4 | EGFR-1.34 | QVQLQESGGGLVQAGGSLRLSCAASGRTFSKYAMGWFRQAPGKEREFVS AISWSDGSTYYADSVKGRFTISRDNAKNTVYLQVNSLKPEDTAVYYCAA TYLVDVWAVHVPIRPYEYDYWGQGTQVTVSS |
| 5 | EGFR-1.38 | QVQLQDSGGGLVQAGDSLRLSCAASGRSFGGYAMGWFRQAPGKEREFVA AISWSGGSTYYADSLKGRFTISRDNAKNTVYLQMNSLKPEDTALYYCAA GLRPSPNYNHERSYDYWGQGTQVTVSS |
| 6 | EGFR-Ia1 | QVQLQESGGGLVQAGGSLLLSCAASGRTFSSYAMGWFRQAPGKEREFVA AINWSGGSTSYADSVKGRFTISRDNTKNTVYLQMNSLKPEDTAAFYCAA TYNPYSRDHYFPRMTTEYDYWGQGTQVTVSS |
| 7 | EGFR-Ia7 | QVQLQESGGRLVQTGGSLRLSCAASGGTFGTYALGWFRQAPGKEREFVA AISRFGSTYYADSVKGRFTISRDNANNTVYLEMNSLKPEDTAVYYCAAR EGVALGLRNDANYWGQGTQVTVSS |
| 8 | EGFR-Ia15 | QVQLQDSGGGLVQAGGSLRLSCAASGGTFSSYAMGWFRQAPGKEREFVA AIGLNTYYADSVKGRFTISRDNAKNTVYLQMNSLKPEDTAVYYCAARTS GVVGGTPKRYDYWGQGTQVTVSS |
| 9 | EGFR-Ia26 | QVQLQESGGGSVQAGGSLKLSCAASGRGFSRYAMGWFRQAPGQDREFVA TISWTNSTDYADSVKGRFAISRDNAKNTAYLQMNSLKPEDTAVYYCAAD KWASSTRSIDYDYWGQGIQVTVSS |
| 10 | EGFR-2.6 | QVQLQESGGGLVQAGGSLRLSCAASGRTFSNYAMGWFRQAPGKEREFVA AINWGGGNTYYADSVKGRFTISRDNAKNTVYLQMNSLKPEDTAVYYCAA SEWGGSDYDHDYDYWGQGTQVTVSS |
| 11 | EGFR-2.20 | EVQLVESGGGLVQAGGSLRLSCAASGRSFSSYAMAWFRQAPGKEREFVA AISWGGGSTYYAVSVKGRFTISRDNAKNTVYLQMNSLKPEDTARYYCAA DETFHSSAYGEYEYWGQGTQVTVSS |
| 12 | EGFR-IIIa5 | EVQLVESGGGLVQAGGSLRLSCTASGRTFSSYAMGWFRQTPGKEREFVA AITSSGGSTYYADSVKGRFTISRDNAKSTMYLQMDSLMLDDTSVYYCAA DSSRPQYSDSALRRILSLSNSYPYWGQGTQVTVSS |
| 13 | EGFR-3.18 | EVQLVESGGGLVQPGGSLRLSCVASGFTFADYAMSWVRQAPGKGLQWVS |
| | | SISYNGDTTYYAESMKDRFTISRDNAKNTLYLQMNSLKSEDTAVYYCAS SGSYYPGHFESWGQGTQVTVSS |
| 14 | EGFR-3.32 | QVQLQESGGGLVQAGGSLRLSCAASGRTFSGYAMGWFRQAPGEEREFVA AISWRGTSTYYGDSAKGRFTISRDNAKNTVYLQMNSLKPEDTAVYYCAA GSHSDYAPDYDYWGQGTQVTVSS |
| 15 | EGFR-3.34 | QVQLQESGGGLVQAGGSLRLSCAASGRTFSSYAIGWFRQAPGKEREFVA AISWGGSNTYYADSVKGRFTISRDNAKNTVYLQMNSLKPEDTAVYYCAA GEVSNSDYAYEYDYWGQGTQVTVSS |
| 16 | EGFR-3.39 | QVQLQESGGGLVQTGGSLRLSCAASGRYIMGWFRQAPGKEREFVAGISR SGASTAYADSVKDRFTISRDSALNTVYLQMNSLKAEDTAVYFCAAALAI RLGIPRGETEYEYWGQGTQVTVSS |
| 17 | EGFR-3.40 | QVKLEESGGGLVQAGGSLRLSCSASGLTFSNYAMAWFRQAPGKEREFVA TISQRGGMRHYLDSVKDRFTISRDNAKNTVYLQMNSLKPDDTAVYYCAA DLMYGVDRRYDYWGRGTQVTVSS |
| 18 | EGFR-4.11 | QVKLEESGGGLVQAGDSLRLSCAASGRSFSSITMGWFRQAPGKERQFVS AINSNGNRYYADSVKGRFTISRDNAKNTVYLQMNSLKPEDTAVYYCAAV QAYSSSSDYYSQEGAYDYWGQGTQVTVSS |
| 19 | EGFR-4.21 | EVQLVESGGGLVQAGGSLRLSCAVSGRTFSSMGWFRQAPGKEREFVATI NLSGDRTDYADSVKGRFTISRDNPKNTVYLQMDSLEPEDSAVYYCAGTS LYPSNLRYYTLPGTYADWGQGTQVTVSS |
| 20 | EGFR-4.22 | QVKLEESGGGLVQAGGSLRLSCAASGSIFSINAMGWYRQAPGKQRELVA RITGTGTGITGAVSTNYADSVKGRFTISRDNARNTVYLQMNSLKPEDTA VYYCAADRSRTIVVPDYWGQGTQVTVSS |
| 21 | EGFR-B11 | QVQLQDSGGGLVQAGGSLRLSCAASRFSSAQYAIGWFRQAPGKEREGVS YITFSGGPTGYADSVKGRFTVSRDNAKNTVYLQMNSLKPEDTAVYYCAA RPYTRPGSMWVSSSLYDNWGQGTQVTVSS |
| 22 | EGFR-F11 | QVQLQESGGRLVQAGGSLRLSCAASEHTFRGYAIGWFRQAPGKEREFVS SITYDGTLTNYADSVTGRFTISRDNAKNTVYLQMNSLKPEDTAVYVCAA GYSYRYTTLNQYDSWGQGTQVTVSS |
| | | Anti-mouse serum albumin |
| 23 | MSA21 | QVQLQESGGGLVQPGGSLRLSCEASGFTFSRFGMTWVRQAPGKGVEWVS GISSLGDSTLYADSVKGRFTISRDNAKNTLYLQMNSLKPEDTAVYYCTI GGSLNPGGQGTQVTVSS |
| 24 | MSA24 | QVQLQESGGGLVQPGNSLRLSCAASGFTFRNFGMSWVRQAPGKEPEWVS SISGSGSNTIYADSVKDRFTISRDNAKSTLYLQMNSLKPEDTAVYYCTI GGSLSRSSQGTQVTVSS |
| 25 | MSA210 | QVQLQESGGGLVQPGGSLRLTCTASGFTFSSFGMSWVRQAPGKGLEWVS AISSDSGTKNYADSVKGRFTISRDNAKKMLFLQMNSLRPEDTAVYYCVI GRGSPSSQGTQVTVSS |
| 26 | MSA212 | QVQLQESGGGLVQPGGSLRLTCTASGFTFRSFGMSWVRQAPGKGLEWVS AISADGSDKRYADSVKGRFTISRDNGKKMLTLDMNSLKPEDTAVYYCVI GRGSPASQGTQVTVSS |
| 41 | MSAcl6 | AVQLVESGGGLVQAGDSLRLSCVVSGTTFSSAAMGWFRQAPGKEREFVG AIKWSGTSTYY TDSVKGRFTISRDNVKNTVYLQMNNLKPEDTGVYTCAADRDRYRDRMGP MTTTDFRFWGQ GTQVTVSS |
| 42 | MSAcl1 2 | QVKLEESGGGLVQTGGSLRLSCAASGRTFSSFAMGWFRQAPGREREFVA SIGSSGITTNY ADSVKGRFTISRDNAKNTVYLQMNSLKPEDTGLCYCAVNRYGIPYRSGT QYQNWGQGTQV TVSS |
| 43 | MSAcl1 0 | EVQLEESGGGLVQPGGSLRLSCAASGLTFNDYAMGWYRQAPGKERDMVA TISIGGRTYYA |
| | | DSVKGRFTISRDNAKNTVYLQMNSLKPEDTAIYYCVAHRQTVVRGPYLL WGQGTQVTVSS |
| 44 | MSAcl1 4 | QVQLVESGGKLVQAGGSLRLSCAASGRTFSNYAMGWFRQAPGKEREFVA GSGRSNSYNYY SDSVKGRFTISRDNAKNTVYLQMNSLKPEDTAVYYCAASTNLWPRDRNL YAYWGQGTQVT VSS |
| 45 | MSAcl1 6 | EVQLVESGGGLVQAGDSLRLSCAASGRSLGIYRMGWFRQVPGKEREFVA AISWSGGTTRY LDSVKGRFTISRDSTKNAVYLQMNSLKPEDTAVYYCAVDSSGRLYWTLS TSYDYWGQGTQ VTVSS |
| 46 | MSAcl1 9 | QVQLVEFGGGLVQAGDSLRLSCAASGRSLGIYKMAWFRQVPGKEREFVA AISWSGGTTRY IDSVKGRFTLSRDNTKNMVYLQMNSLKPDDTAVYYCAVDSSGRLYWTLS TSYDYWGQGTQ VTVSS |
| 47 | MSAcl5 | EVQLVESGGGLVQAGGSLSLSCAASGRTFSPYTMGWFRQAPGKEREFLA GVTWSGSSTFY GDSVKGRFTASRDSAKNTVTLEMNSLNPEDTAVYYCAAAYGGGLYRDPR SYDYWGRGTQV TVSS |
| 48 | MScl11 | AVQLVESGGGLVQAGGSLRLSCAASGFTLDAWPIAWFRQAPGKEREGVS CIRDGTTYYAD SVKGRFTISSDNANNTVYLQTNSLKPEDTAVYYCAAPSGPATGSSHTFG IYWNLRDDYDN WGQGTQVTVSS |
| 49 | MSAcl1 5 | EVQLVESGGGLVQAGGSLRLSCAASGFTFDHYTIGWFRQVPGKEREGVS CISSSDGSTYY ADSVKGRFTISSDNAKNTVYLQMNTLEPDDTAVYYCAAGGLLLRVEELQ ASDYDYWGQGI QVTVSS |
| 50 | MSAcl8 | AVQLVDSGGGLVQPGGSLRLSCTASGFTLDYYAIGWFRQAPGKEREGVA CISNSDGSTYY GDSVKGRFTISRDNAKTTVYLQMNSLKPEDTAVYYCATADRHYSASHHP FADFAFNSWGQ GTQVTVSS |
| 51 | MSAcl7 | EVQLVESGGGLVQAGGSLRLSCAAYGLTFWRAAMAWFRRAPGKERELVV ARNWGDGSTRY ADSVKGRFTISRDNAKNTVYLQMNSLKPEDTAVYYCAAVRTYGSATYDI WGQGTQVTVSS |
| 52 | MSAcl2 0 | EVQLVESGGGLVQDGGSLRLSCIFSGRTFANYAMGWFRQAPGKEREFVA AINRNGGTTNY ADALKGRFTISRDNTKNTAFLQMNSLKPDDTAVYYCAAREWPFSTIPSG WRYWGQGTQVT VSS |
| 53 | MSAcl4 | DVQLVESGGGWVQPGGSLRLSCAASGPTASSHAIGWFRQAPGKEREFVV GINRGGVTRDY ADSVKGRFAVSRDNVKNTVYLQMNRLKPEDSAIYICAARPEYSFTAMSK GDMDYWGKGTL VTVSS |
| | | |
| | | |
| | | Anti-mouse serum albumin/anti EGFR |
| 27 | MSA21/EGFR-1.4 | QVQLQESGGGLVQPGGSLRLSCEASGFTFSRFGMTWVRQAPGKGVEWVS GISSLGDSTLYADSVKGRFTISRDNAKNTLYLQMNSLKPEDTAVYYCTI GGSLNPGGQGTQVTVSSEPKTPKPQPAAAEVQLVESGGGLVQAGGSLRL SCAASGRTFSNYVMGWFRQAPGKERDFVVGIGRSGGDNTYYADSVKGRF TISWDNAKNTMYLQMNSLKPEDTAVYYCAASTYSRDTIFTKWANYNYWG QGTQVTVSS |
| 28 | MSA24/EGFR-1.9 | QVQLQESGGGLVQPGNSLRLSCAASGFTFRNFGMSWVRQAPGKEPEWVS SISGSGSNTIYADSVKDRFTISRDNAKSTLYLQMNSLKPEDTAVYYCTI GGSLSRSSQGTQVTVSSEPKTPKPQPAAAQVQLQESGGGLVKAGGSLRL SCAASGRTFSSYVMGWFRQAPGKEREFVGAIHWSGGRTYYADSVKGRFT ISSDNAKNTLYLQMNSLKPEDTAVYYCAASRIIYSYVNYVNPGEYDYWG QGTQVTVSS |
| 29 | MSA21/EGFR-1.33 | QVQLQESGGGLVQPGGSLRLSCEASGFTFSRFGMTWVRQAPGKGVEWVS GISSLGDSTLYADSVKGRFTISRDNAKNTLYLQMNSLKPEDTAVYYCTI GGSLNPGGQGTQVTVSSEPKTPKPQPAAAEVQLVESGGGLVQPGGSLRL SCAASGFTFSSHYMSWFRQAPGKEREFVAAITSSSRTYYTESVKGRFTI SRDNAKNTVYLQMNSLKSEDTAVYYCAADRTFYGSTWSKYDYRGQGTQV TVSS |
| 30 | MSA24/EGFR-1.33 | QVQLQESGGGLVQPGNSLRLSCAASGFTFRNFGMSWVRQAPGKEPEWVS SISGSGSNTIYADSVKDRFTISRDNAKSTLYLQMNSLKPEDTAVYYCTI GGSLSRSSQGTQVTVSSEPKTPKPQPAAAEVQLVESGGGLVQPGGSLRL SCAASGFTFSSHYMSWFRQAPGKEREFVAAITSSSRTYYTESVKGRFTI SRDNAKNTVYLQMNSLKSEDTAVYYCAADRTFYGSTWSKYDYRGQGTQV TVSS |
| 31 | MSA210/EGFR-1.33 | QVQLQESGGGLVQPGGSLRLTCTASGFTFSSFGMSWVRQAPGKGLEWVS AISSDSGTKNYADSVKGRFTISRDNAKKMLFLQMNSLRPEDTAVYYCVI GRGSPSSQGTQVTVSSEPKTPKPQPAAAEVQLVESGGGLVQPGGSLRLS CAASGFTFSSHYMSWFRQAPGKEREFVAAITSSSRTYYTESVKGRFTIS RDNAKNTVYLQMNSLKSEDTAVYYCAADRTFYGSTWSKYDYRGQGTQVT VSS |
| 32 | MSA212/EGFR-1.33 | QVQLQESGGGLVQPGGSLRLTCTASGFTFRSFGMSWVRQAPGKGLEWVS AISADGSDKRYADSVKGRFTISRDNGKKMLTLDMNSLKPEDTAVYYCVI GRGSPASQGTQVTVSSEPKTPKPQPAAAEVQLVESGGGLVQPGGSLRLS CAASGFTFSSHYMSWFRQAPGKEREFVAAITSSSRTYYTESVKGRFTIS RDNAKNTVYLQMNSLKSEDTAVYYCAADRTFYGSTWSKYDYRGQGTQVT VSS |
| 33 | MSA21/EGFR-Ia1 | QVQLQESGGGLVQPGGSLRLSCEASGFTFSRFGMTWVRQAPGKGVEWVS GISSLGDSTLYADSVKGRFTISRDNAKNTLYLQMNSLKPEDTAVYYCTI GGSLNPGGQGTQVTVSSEPKTPKPQPAAAQVQLQESGGGLVQAGGSLLL SCAASGRTFSSYAMGWFRQAPGKEREFVAAINWSGGSTSYADSVKGRFT ISRDNTKNTVYLQMNSLKPEDTAAFYCAATYNPYSRDHYFPRMTTEYDY WGQGTQVTVSS |
| 34 | MSA21/EGFR-Ia7 | QVQLQESGGGLVQPGGSLRLSCEASGFTFSRFGMTWVRQAPGKGVEWVS GISSLGDSTLYADSVKGRFTISRDNAKNTLYLQMNSLKPEDTAVYYCTI GGSLNPGGQGTQVTVSSEPKTPKPQPAAAQVQLQESGGRLVQTGGSLRL SCAASGGTFGTYALGWFRQAPGKEREFVAAISRFGSTYYADSVKGRFTI SRDNANNTVYLEMNSLKPEDTAVYYCAAREGVALGLRNDANYWGQGTQV TVSS |
| 35 | MSA21/EGFR-Ia15 | QVQLQESGGGLVQPGGSLRLSCEASGFTFSRFGMTWVRQAPGKGVEWVS GISSLGDSTLYADSVKGRFTISRDNAKNTLYLQMNSLKPEDTAVYYCTI GGSLNPGGQGTQVTVSSEPKTPKPQPAAAQVQLQDSGGGLVQAGGSLRL SCAASGGTFSSYAMGWFRQAPGKEREFVAAIGLNTYYADSVKGRFTISR DNAKNTVYLQMNSLKPEDTAVYYCAARTSGVVGGTPKRYDYWGQGTQVT VSS |
| 36 | MSA21/EGFR-Ia26 | QVQLQESGGGLVQPGGSLRLSCEASGFTFSRFGMTWVRQAPGKGVEWVS GISSLGDSTLYADSVKGRFTISRDNAKNTLYLQMNSLKPEDTAVYYCTI GGSLNPGGQGTQVTVSSEPKTPKPQPAAAQVQLQESGGGSVQAGGSLKL SCAASGRGFSRYAMGWFRQAPGQDREFVATISWTNSTDYADSVKGRFAI SRDNAKNTAYLQMNSLKPEDTAVYYCAADKWASSTRSIDYDYWGQGIQV TVSS |
| 37 | MSA21/EGFR-2.6 | QVQLQESGGGLVQPGGSLRLSCEASGFTFSRFGMTWVRQAPGKGVEWVS GISSLGDSTLYADSVKGRFTISRDNAKNTLYLQMNSLKPEDTAVYYCTI GGSLNPGGQGTQVTVSSEPKTPKPQPAAAQVQLQESGGGLVQAGGSLRL SCAASGRTFSNYAMGWFRQAPGKEREFVAAINWGGGNTYYADSVKGRFT ISRDNAKNTVYLQMNSLKPEDTAVYYCAASEWGGSDYDHDYDYWGQGTQ VTVSS |
| 38 | MSA21/EGFR-4.22 | QVQLQESGGGLVQPGGSLRLSCEASGFTFSRFGMTWVRQAPGKGVEWVS GISSLGDSTLYADSVKGRFTISRDNAKNTLYLQMNSLKPEDTAVYYCTI GGSLNPGGQGTQVTVSSEPKTPKPQPAAAQVKLEESGGGLVQAGGSLRL SCAASGSIFSINAMGWYRQAPGKQRELVARITGTGTGITGAVSTNYADS VKGRFTISRDNARNTVYLQMNSLKPEDTAVYYCAADRSRTIVVPDYWGQ GTQVTVSS |
| 39 | MSA21/EGFR-B11 | QVQLQESGGGLVQPGGSLRLSCEASGFTFSRFGMTWVRQAPGKGVEWVS GISSLGDSTLYADSVKGRFTISRDNAKNTLYLQMNSLKPEDTAVYYCTI GGSLNPGGQGTQVTVSSEPKTPKPQPAAAQVQLQDSGGGLVQAGGSLRL SCAASRFSSAQYAIGWFRQAPGKEREGVSYITFSGGPTGYADSVKGRFT VSRDNAKNTVYLQMNSLKPEDTAVYYCAARPYTRPGSMWVSSLYDNWGQ GTQVTVSS |
| 40 | MSA21/EGFR-F11 | QVQLQESGGGLVQPGGSLRLSCEASGFTFSRFGMTWVRQAPGKGVEWVS GISSLGDSTLYADSVKGRFTISRDNAKNTLYLQMNSLKPEDTAVYYCTI GGSLNPGGQGTQVTVSSEPKTPKPQPAAAQVQLQESGGRLVQAGGSLRL SCAASEHTFRGYAIGWFRQAPGKEREFVSSITYDGTLTNYADSVTGRFT ISRDNAKNTVYLQMNSLKPEDTAVYVCAAGYSYRYTTLNQYDSWGQGTQ VTVSS |

**Table 6**

| **Seq ID** | **Name** | **Sequence 5' - 3'** |
|---|---|---|
| 54 | ABL002 | GGCTGAGCTCGGTGGTCCTGGCT |
| 55 | ABL010 | AACTGGAAGAATTCGCGGCCGCAGGAATTTTTTTTTTTTTTTTTT |
| 56 | ABL037 | CATGCCATGACTCGCGGCCCAGCCGGCCATGGCCGAGGTGCAGCTGGTGGA GTCTGG |
| 57 | ABL038 | CATGCCATGACTCGCGGCCCAGCCGGCCATGGCCGATGTGCAGCTGGTGGA GTCTGG |
| 58 | ABL039 | CATGCCATGACTCGCGGCCCAGCCGGCCATGGCCGCGGTGCAGCTGGTGGA GTCTGG |
| 59 | ABL040 | CATGCCATGACTCGCGGCCCAGCCGGCCATGGCCGCCGTGCAGCTGGTGGA TTCTGG |
| 60 | ABL041 | CATGCCATGACTCGCGGCCCAGCCGGCCATGGCCCAGGTGCAGCTGGTGGA GTCTGG |
| 61 | ABL042 | CATGCCATGACTCGCGGCCCAGCCGGCCATGGCCCAGGTACAGCTGGTGGA GTCTGG |
| 62 | ABL043 | CATGCCATGACTCGCGGCCCAGCCGGCCATGGCCCAGGTAAAGCTGGAGGA GTCTGG |
| 63 | geneIII | CCACAGACAGCCCTCATAG |
| 64 | M13 rev | GGATAACAATTTCACACAGG |

## Claims

1. An anti-EGFR polypeptide comprising at least one single domain antibody directed against EGFR, wherein said single domain antibody is derived from a heavy chain antibody naturally devoid of light chain (VHH) and wherein said at least one single domain antibody is able to internalize EGFR on EGFR⁺ cells.

2. Anti-EGFR polypeptide according to claim 1, obtainable by incubating recombinant phages expressing a repertoir of cloned VHHs with EGFR⁺ cells under conditions to allow internalization of the phages and subsequent release of the internalized phages.

3. Anti-EGFR polypeptide according to any of claims I or 2, in which the EGFR⁺ cell is selected from HER-14 and A431 cells.

4. Anti-EGFR polypeptide according to any of claims 1 to 3, wherein the at least one single domain antibody corresponds to a sequence represented by any of SEQ ID NOs: 21, 22 or
a) an homologous sequence of any of SEQ ID NOs: 21, 22 with a sequence identity of more than 70% with the parent sequence;
b) a functional portion of any of SEQ ID NOs: 21, 22 that maintains the interaction with the target with affinity of 1 x 10⁻⁶ M or better,
c) a functional portion of any of SEQ ID NOs: 21, 22 that comprises a partial deletion of the complete amino acid sequence and still maintains the binding site(s) and protein domain(s) necessary for the binding of and interaction with EGFR.

5. Anti-EGFR polypeptide according to any of claims 1 to 4, wherein the at least one single domain antibody corresponds to a sequence represented by any of SEQ ID NOs: 21, 22, or to an homologous sequence of any of SEQ ID NOs: 21, 22 with a sequence identity of more than 80% with the parent sequence.

6. Anti-EGFR polypeptide according to any of claims 1 to 5, wherein the at least one single domain antibody corresponds to a sequence represented by any of SEQ ID NOs: 21, 22, or to an homologous sequence of any of SEQ ID NOs: 21, 22 with a sequence identity of more than 90% with the parent sequence.

7. Anti-EGFR polypeptide according to any of claims 1 to 6, wherein the at least one single domain antibody competes for or inhibits binding of the natural ligand to EGFR

8. Anti-EGFR polypeptide according to claim 7, wherein said natural ligand is EGF.

9. Anti-EGFR polypeptide according to any of claims 1 to 8, wherein the at least one single domain antibody is capable of binding to its target with an affinity of better than 10⁻⁶ M.

10. Anti-EGFR polypeptide according to any of claims 1 to 9, further comprising at least one single domain antibody directed against one or more serum proteins.

11. Anti-EGFR polypeptide according to claim 10, wherein said at least one single domain antibody directed against one or more serum proteins is directed against any of serum albumin, serum immunoglobulins, thyroxine-binding protein, transferrin, or fibrinogen or a fragment thereof.

12. Anti-EGFR polypeptide according to any of claims 1 to 11, in which the at least one single domain antibody is a VHH domain.

13. Anti-EGFR polypeptide according to claim 12, in which the at least one single domain antibody is a VHH domain carrying an amino acid from the group consisting of glycine, alanine, valine, leucine, isoleucine, proline, phenylalanine, tyrosine, tryptophan, methionine, serine, threonine, asparagine, or glutamine at position 45 according to the Kabat numbering.

14. Anti-EGFR polypeptide according to claim 12 in which the at least one single domain antibody is a VHH domain that carries an arginine residue at position 103 according to the Kabat numbering.

15. Anti-EGFR polypeptide according to any of claims 12 to 14, in which said VHH domain is obtained by immunizing a camel and obtaining hybridoma's therefrom, or by cloning a library of single domain antibodies and subsequent selection by using phage display.

16. Anti-EGFR polypeptide according to any of claims 1 to 15, in which the at least one single domain antibody is a humanized VHH.

17. Anti-EGFR polypeptide according to claim 16, in which the at least one single domain antibody is humanized by replacing one or more of the *Camelidae* amino acids by their human counterpart as found in the human consensus sequence.

18. Anti-EGFR polypeptide according to any of claims 16 or 17, in which the at least one single domain antibody is humanized by replacing any of the following residues either alone or in combination: FR1 positions 1, 5, 28 and 30, the hallmark amino acid at position 44 and 45 in FR2, FR3 residues 74, 75, 76, 83, 84, 93 and 94 and positions 103, 104, 108 and 111 in FR4; numbering according to the Kabat numbering.

19. Anti-EGFR polypeptide according to any of claims 1 to 18, further comprising at least one single domain antibody selected from the group consisting of anti-IFN-gamma single domain antibody, anti-TNF-alpha single domain antibody, anti-TNF-alpha receptor single domain antibody and anti-IFN-gamma receptor single domain antibody.

20. Anti-EGFR polypeptide according to any of claims 1 to 19, wherein the number of single domain antibodies directed against EGFR is at least two.

21. Anti-EGFR polypeptide according to claim 20, in which the two or more single domain antibodies are different in sequence.

22. Anti-EGFR polypeptide according to claim 20, in which the two or more single domain antibodies are identical in sequence.

23. Anti-EGFR polypeptide according to any of claims 19 to 22, in which the two or more single domain antibodies are fused genetically at the DNA level.

24. Anti-EGFR polypeptide according to claim 23, in which the single domain antibodies are linked to each other directly.

25. Anti-EGFR polypeptide according to any of claims 19 to 24, which is a bivalent, trivalent or tetravalent molecule.

26. Anti-EGFR polypeptide according to any of claims 1 to 25, further comprising a carrier that enhances the transfer through the intestinal wall into the bloodstream.

27. Anti-EGFR polypeptide according to claim 26, wherein said carrier is a VHH that binds specifically to a receptor on the intestinal wall.

28. Anti-EGFR polypeptide according to any of claims 1 to 27, further comprising a carrier that enhances transport of the polypeptide from the lung lumen to the blood.

29. Anti-EGFR polypeptide according to claim 28, wherein said carrier is a VHH that binds specifically to a receptor on the mucosal surface.

30. Anti-EGFR polypeptide according to claim 29, wherein said receptor is the Fc receptor N (FcRn).

31. Nucleic acid encoding a polypeptide according to any of claims I to 30.

32. Host cell comprising the nucleic acid according to claim 31.

33. Composition comprising a polypeptide according to any of claims to 30 or a nucleic acid according to claim 31, and a suitable pharmaceutical vehicle.

34. Composition according to claim 33 adapted to the chosen route of administration, *i.e.,* orally, vaginally, rectally, parenterally, intra-nasally, by inhalation, sublingually, intravenous, intramuscular, topical or subcutaneous routes.

35. Composition according to any of claims 33 or 34, suitable for injection or infusion.

36. Therapeutic composition comprising an anti-EGFR polypeptide according to any of claims 1 to 30 in combination with anti-neoplastic or chemotherapeutic agent.

37. Therapeutic composition according to claim 36 for separate administration of the components.

38. Anti-EAFR polypeptide according to any of claims I to 30, nucleic acid according to claim 31, or composition according to any of claims 33 to 37 for use in treating, and/or preventing of disorders relating to inflammatory processes.

39. Anti-EGFR polypeptide according to any of claims 1 to 30, nucleic acid according to claim 31, or composition according to any of claims 33 to 37 for use in treating and/or preventing epithelial cancers, such as lung, liver, central nervous system, bone, blood and lymphatic system, colon, breast, prostate, rectum, bladder, head and neck, ovarian, testis, pancreatic and squamos cell carcinoma.

40. Anti-EGFR polypeptide according to any of claims 1. to 30, nucleic acid according to claim 31, or composition according to any of claims 33 to 37 for use in treating and/or preventing autoimmune diseases, such as Addison's disease (adrenal), Autoimmune diseases of the ear (ear), Autoimmune diseases of the eye (eye), Autoimmune hepatitis (liveer), Autoimmune parotitis (parotid glands), Crohn's disease (intestine), Diabetes Type I (pancreas), Epididymitis (epididymis), Glomerulonephritis (kidneys), Graves' disease (thyroid), Guillain-Barre syndrome (nerve cells), Hashimoto's disease (thyroid), Hemolytic anemia (red blood cells), Systemic lupus erythematosus (multiple tissues), Male infertility (sperm), Multiple sclerosis (nerve cells), Myasthenia Gravis (neuromuscular junction), Pemphigus (primarily skin), Psoriasis (skin), Rheumatic fever (heart and joints), Rheumatoid arthritis (joint lining), Sarcoidosis (multiple tissues and organs), Scleroderma (skin and connective tissues), Sjogren's syndrome (exocrine glands, and other tissues), Spondyloarthropathies (axial skeleton, and other tissues), Thyroiditis (thyroid), Vasculitis (blood vessels).

41. Use of a polypeptide or polypeptide construct according to any of claims 1 to 30 or of a nucleic acid according to claim 31, for the preparation of a medicament for treating a disorder relating to inflammatory processes and cancer.

42. Use of a polypeptide or polypeptide construct according to any of claims 1 to 30 or of a nucleic acid according to claim 31, for the preparation of a medicament for treating, preventing and/or epithelial cancers, such as lung, liver, central nervous system, bone, blood and lymphatic system, colon, breast, prostate, rectum, bladder, head and neck, ovarian, testis, pancreatic and squamos cell carcinoma.

43. Use of a polypeptide or polypeptide construct according to any of claims 1 to 30 or of a nucleic acid according to claim 31, for the preparation of a medicament for treating, and/or preventing autoimmune diseases, such as Addison's disease (adrenal), Autoimmune diseases of the ear (ear), Autoimmune diseases of the eye (eye), Autoimmune hepatitis (liver), Autoimmune parotitis (parotid glands), Crohn's disease (intestine), Diabetes Type 1 (pancreas), Epididymitis (epididymis), Glomerulonephritis (kidneys), Graves' disease (thyroid), Guillain-Barre syndrome (nerve cells), Hashimoto's disease (thyroid), Hemolytic anemia (red blood cells). Systemic lupus erythematosus (multiple tissues), Male infertility (sperm), Multiple sclerosis (nerve cells), Myasthenia Gravis (neuromuscular junction), Pemphigus (primarily skin), Psoriasis (skin), Rheumatic fever (heart and joints), Rheumatoid arthritis (joint lining), Sarcoidosis (multiple tissues and organs), Scleroderma (skin and connective tissues), Sjogren's syndrome (exocrine glands, and other tissues), Spondyloarthropathies (axial skeleton, and other tissues), Thyroiditis (thyroid), Vasculitis (blood vessels).

44. Method of producing a polypeptide according to any of claims 1 to 30, comprising:
(a) culturing host cells comprising nucleic acid capable of encoding a polypeptide or polypeptide construct according to any of claims 1 to 30 under conditions allowing the expression of the polypeptide, and,
(b) recovering the produced polypeptide from the culture.

45. Method according to claim 44 wherein said host cells are bacterial cells, yeast cells, mammalian cells or insect cells.

46. Method according to claim 45, in which said hosts cells are bacterial cells or yeast cells.

47. Method according to claim 46, in which said hosts cells are *E. coli* cells, *S. cerevisiae* cells or *P. pastoris* cells.

48. Method of diagnosing a disorder **characterised by** the dysfunction of EGFR comprising the steps of:
(a) contacting a sample with a polypeptide according to any of claims 1 to 30,
(b) detecting binding of said polypeptide to said sample, and
(c) comparing the binding detected in step (b) with a standard, wherein a difference in binding relative to said sample is diagnostic of a disorder **characterised by** dysfunction of EGFR.

49. Kit for screening for a disorder **characterised by** dysfunction of EGFR comprising a polypeptide according to any of claims 1 to 30.

## Patentansprüche

1. Anti-EGFR-Polypeptid, umfassend wenigstens einen gegen EGFR gerichteten Einzeldomänenantikörper, wobei der Einzeldomänenantikörper von einem Schwere-Ketten-Antikörper abgeleitet ist, der von Natur aus keine leichte Kette aufweist (VHH), und wobei der wenigstens eine Einzeldomänenantikörper in der Lage ist, EGFR auf EGFR⁺-Zellen zu internalisieren.

2. Anti-EGFR-Polypeptid gemäß Anspruch 1, erhältlich durch Inkubieren von rekombinanten Phagen, die ein Repertoire von klonierten VHHs exprimieren, mit EGFR⁺-Zellen unter Bedingungen, die eine Internalisierung der Phagen und eine anschließende Freisetzung der internalisierten Phagen ermöglichen.

3. Anti-EGFR-Polypeptid gemäß Anspruch 1 oder 2, wobei die EGFR⁺-Zelle ausgewählt ist aus HER-14- und A431-Zellen.

4. Anti-EGFR-Polypeptid gemäß einem der Ansprüche 1 bis 3, wobei der wenigstens eine Einzeldomänenantikörper einer Sequenz entspricht, die durch eine der SEQ ID Nrn.: 21, 22 dargestellt ist, oder
a) einer homologen Sequenz von einer der SEQ ID Nrn.: 21, 22 mit einer Sequenzidentität von mehr als 70 % mit der Stammsequenz;
b) einem funktionellen Teil von einer der SEQ ID Nrn.: 21, 22, der die Wechselwirkung mit dem Ziel mit einer Affinität von 1 x 10⁻⁶ M oder besser beibehält;
c) einem funktionellen Teil von einer der SEQ ID Nrn.: 21, 22, der eine teilweise Deletion der vollständigen Aminosäuresequenz umfasst und trotzdem die Bindungsstelle(n) und Proteindomäne(n), die für das Binden von und die Wechselwirkung mit EGFR notwendig ist (sind), beibehält;

5. Anti-EGFR-Polypeptid gemäß einem der Ansprüche 1 bis 4, wobei der wenigstens eine Einzeldomänenantikörper einer Sequenz, die durch eine der SEQ ID Nrn.: 21, 22 dargestellt ist, oder einer homologen Sequenz von einer der SEQ ID Nrn.: 21, 22 mit einer Sequenzidentität von mehr als 80 % mit der Stammsequenz entspricht.

6. Anti-EGFR-Polypeptid gemäß einem der Ansprüche 1 bis 5, wobei der wenigstens eine Einzeldomänenantikörper einer Sequenz, die durch eine der SEQ ID Nrn. : 21, 22 dargestellt ist, oder einer homologen Sequenz einer der SEQ ID Nrn.: 21, 22 mit einer Sequenzidentität von mehr als 90 % mit der Stammsequenz entspricht.

7. Anti-EGFR-Polypeptid gemäß einem der Ansprüche 1 bis 6, wobei der wenigstens eine Einzeldomänenantikörper um EGFR konkurriert oder das Binden des natürlichen Liganden an EGFR inhibiert.

8. Anti-EGFR-Polypeptid gemäß Anspruch 7, wobei der natürliche Ligand EGF ist.

9. Anti-EGFR-Polypeptid gemäß einem der Ansprüche 1 bis 8, wobei der wenigstens eine Einzeldomänenantikörper in der Lage ist, sein Ziel mit einer Affinität von mehr als 10⁻⁶ M zu binden.

10. Anti-EGFR-Polypeptid gemäß einem der Ansprüche 1 bis 9, ferner umfassend wenigstens einen gegen ein oder mehrere Serumproteine gerichteten Einzeldomänenantikörper.

11. Anti-EGFR-Polypeptid gemäß Anspruch 10, wobei der gegen ein oder mehrere Serumproteine gerichtete Einzeldomänenantikörper gegen einen von Serumalbumin, Serumimmunglobulinen, Thyroxin-bindendem Protein, Transferrin oder Fibrinogen oder einem Fragment davon gerichtet ist.

12. Anti-EGFR-Polypeptid gemäß einem der Ansprüche 1 bis 11, in dem der wenigstens eine Einzeldomänenantikörper eine VHH-Domäne ist.

13. Anti-EGFR-Polypeptid gemäß Anspruch 12, in dem der wenigstens eine Einzeldomänenantikörper eine VHH-Domäne ist, die eine Aminosäure aus der Gruppe, bestehend aus Glycin, Alanin, Valin, Leucin, Isoleucin, Prolin, Phenylalanin, Tyrosin, Tryptophan, Methionin, Serin, Threonin, Asparagin oder Glutamin, an Position 45 gemäß der Kabat-Nummerierung trägt.

14. Anti-EGFR-Polypeptid gemäß Anspruch 12, in dem der wenigstens eine Einzeldomänenantikörper eine VHH-Domäne ist, die einen Argininrest an Position 103 gemäß der Kabat-Nummerierung trägt.

15. Anti-EGFR-Polypeptid gemäß einem der Ansprüche 12 bis 14, in dem die VHH-Domäne durch Immunisierung eines Kamels und Erhalten von Hybridoma davon oder durch Klonieren einer Bibliothek von Einzeldomänenantikörpern und anschließender Selektion mittels eines Phagen-Displays erhältlich ist.

16. Anti-EGFR-Polypeptid gemäß einem der Ansprüche 1 bis 15, in dem der wenigstens eine Einzeldomänenantikörper ein humanisierter VHH ist.

17. Anti-EGFR-Polypeptid gemäß Anspruch 16, in dem der wenigstens eine Einzeldomänenantikörper durch Ersetzen einer oder mehrerer der Camelidae-Aminosäuren durch deren humanes Gegenstück, wie es in der humanen Konsensus-Sequenz zu finden ist, humanisiert ist.

18. Anti-EGFR-Polypeptid gemäß Anspruch 16 oder 17, in dem der wenigstens eine Einzeldomänenantikörper durch Ersetzen eines der folgenden Reste, entweder alleine oder in Kombination, humanisiert ist: FR1-Positionen 1, 5, 28 und 30, die Kennzeichenaminosäure an Position 44 und 45 in FR2, FR3-Reste 74, 75, 76, 83, 84, 93 und 94 und Positionen 103, 104, 108 und 111 in FR4; Nummerierung gemäß der Kabat-Nummerierung.

19. Anti-EGFR-Polypeptid gemäß einem der Ansprüche 1 bis 18, ferner umfassend wenigstens einen Einzeldomänenantikörper ausgewählt aus der Gruppe, bestehend aus Anti-IFN-gamma-Einzeldomänenantikörper, Anti-TNF-alpha-Einzeldomänenantikörper, Anti-TNF-alpha-Rezeptor-Einzeldomänenantikörper und Anti-IFN-gamma-Rezeptor-Einzeldomänenantikörper.

20. Anti-EGFR-Polypeptid gemäß einem der Ansprüche 1 bis 19, wobei die Anzahl an Einzeldomänenantikörpern, die gegen EGFR gerichtete sind, wenigstens zwei ist.

21. Anti-EGFR-Polypeptid gemäß Anspruch 20, in dem die zwei oder mehr Einzeldomänenantikörper in der Sequenz unterschiedlich sind.

22. Anti-EGFR-Polypeptid gemäß Anspruch 20, in dem die zwei oder mehr Einzeldomänenantikörper in der Sequenz identisch sind.

23. Anti-EGFR-Polypeptid gemäß einem der Ansprüche 19 bis 22, in dem die zwei oder mehr Einzeldomänenantikörper auf der DNA-Ebene genetisch fusioniert sind.

24. Anti-EGFR-Polypeptid gemäß Anspruch 23, in dem die Einzeldomänenantikörper direkt miteinander verknüpft sind.

25. Anti-EGFR-Polypeptid gemäß einem der Ansprüche 19 bis 24, der ein bivalentes, trivalentes oder tetravalentes Molekül ist.

26. Anti-EGFR-Polypeptid gemäß einem der Ansprüche 1 bis 25, ferner umfassend einen Träger, der den Übertritt durch die Darmwand in den Blutstrom verstärkt.

27. Anti-EGFR-Polypeptid gemäß Anspruch 26, wobei der Träger ein VHH ist, der spezifisch an einen Rezeptor auf der Darmwand bindet.

28. Anti-EGFR-Polypeptid gemäß einem der Ansprüche 1 bis 27, ferner umfassend einen Träger, der den Transport des Polypeptids vom Lungenlumen zum Blut verstärkt.

29. Anti-EGFR-Polypeptid gemäß Anspruch bis 28, wobei der Träger ein VHH ist, der spezifisch an einen Rezeptor auf der Schleimhautoberfläche bindet.

30. Anti-EGFR-Polypeptid gemäß Anspruch bis 29, wobei der Rezeptor der Fc-Rezeptor (FcRn) ist.

31. Nukleinsäure, kodierend für ein Polypeptid gemäß einem der Ansprüche 1 bis 30.

32. Wirtszelle, umfassend die Nukleinsäure gemäß Anspruch 31.

33. Zusammensetzung, umfassend ein Polypeptid gemäß einem der Ansprüche 1 bis 30 oder eine Nukleinsäure gemäß Anspruch 31 und einen geeigneten pharmazeutischen Träger.

34. Zusammensetzung gemäß Anspruch 33, angepasst an den gewählten Verabreichungsweg, d.h., orale, vaginale, rektale, parenterale, intranasale, durch Inhalation, sublinguale, intravenöse, intramuskuläre, topische oder subkutane Wege.

35. Zusammensetzung gemäß Anspruch 33 oder 34, die zur Injektion oder Infusion geeignet ist.

36. Therapeutische Zusammensetzung, umfassend ein Anti-EGFR-Polypeptid gemäß einem der Ansprüche 1 bis 30 in Kombination mit einem anti-neoplastischen oder chemotherapeutischen Mittel.

37. Therapeutische Zusammensetzung gemäß Anspruch 36 zur separaten Verabreichung der Bestandteile.

38. Anti-EGFR-Polypeptid gemäß einem der Ansprüche 1 bis 30, Nukleinsäure gemäß Anspruch 31 oder Zusammensetzung gemäß einem der Ansprüche 33 bis 37 zur Verwendung in der Behandlung und/oder Vorbeugung von Störungen, die mit entzündlichen Prozessen im Zusammenhang stehen.

39. Anti-EGFR-Polypeptid gemäß einem der Ansprüche 1 bis 30, Nukleinsäure gemäß Anspruch 31 oder Zusammensetzung gemäß einem der Ansprüche 33 bis 37 zur Verwendung in der Behandlung und/oder Vorbeugung von epithelialen Karzinomen, wie Karzinomen der Lunge, der Leber, des zentralen Nervensystems, des Knochens, des Blut- und lymphatischen Systems, des Darms, der Brust, der Prostata, des Rektums, der Blase, des Kopfs und Nackens, der Ovarien, der Hoden, der Bauchspeicheldrüse und des Plattenepithels.

40. Anti-EGFR-Polypeptid gemäß einem der Ansprüche 1 bis 30, Nukleinsäure gemäß Anspruch 31 oder Zusammensetzung gemäß einem der Ansprüche 33 bis 37 zur Verwendung in der Behandlung und/oder Vorbeugung von Autoimmunerkrankungen, wie Morbus Addison (Nebenniere), Autoimmunerkrankung des Ohrs (Ohr), Autoimmunerkrankung des Auges (Auge), Autoimmunhepatits (Leber), Autoimmunparotitis (Glandula parotis), Morbus Crohn (Darm), Diabetes Typ I (Bauchspeicheldrüse), Epididymitis (Epididymis), Glomerulonephritis (Nieren), Basedow'sche Erkrankung (Schilddrüse), Guillain-Barre-Syndrom (Nervenzellen), Hashimoto-Erkrankung (Schilddrüse), hämolytische Anämie (rote Blutzellen), systemischer Lupus erythematodes (mehrere Gewebe), männliche Unfruchtbarkeit (Spermium), Multiple Sklerose (Nervenzellen) Myasthenia Gravis (neuromuskuläre Synapse), Pemphigus (hauptsächlich Haut), Psoriasis (Haut), rheumatisches Fieber (Herz und Gelenke), rheumatoide Arthritis (Gelenkkapsel), Sarcoidose (mehrere Gewebe und Organe), Sklerodermie (Haut und Bindegewebe), Sjogren-Syndrom (exokrine Drüsen und andere Gewebe), Spondyloarthropathien (Axialskelett und andere Gewebe), Thyroiditis (Schilddrüse), Gefäßentzündung (Blutgefäße).

41. Verwendung eines Polypeptids oder Polypeptidkonstrukts gemäß einem der Ansprüche 1 bis 30 oder einer Nukleinsäure gemäß Anspruch 31 zur Herstellung eines Medikaments zur Behandlung einer Störung, die mit entzündlichen Prozessen in Zusammenhang steht, und Krebs.

42. Verwendung eines Polypeptids oder Polypeptidkonstrukts gemäß einem der Ansprüche 1 bis 30 oder einer Nukleinsäure gemäß Anspruch 31 zur Herstellung eines Medikaments zur Behandlung und/oder Vorbeugung von epithelialen Karzinomen, wie Karzinomen der Lunge, der Leber, des zentralen Nervensystems, des Knochens, des Blut- und lymphatischen Systems, des Darms, der Brust, der Prostata, des Rektums, der Blase, des Kopfs und Nackens, der Ovarien, der Hoden, der Bauchspeicheldrüse und des Plattenepithels.

43. Verwendung eines Polypeptids oder Polypeptidkonstrukts gemäß einem der Ansprüche 1 bis 30 oder einer Nukleinsäure gemäß Anspruch 31 zur Herstellung eines Medikaments zur Behandlung und/oder Vorbeugung von Autoimmunerkrankungen, wie Morbus Addison (Nebenniere), Autoimmunerkrankung des Ohrs (Ohr), Autoimmunerkrankung des Auges (Auge), Autoimmunhepatits (Leber), Autoimmunparotitis (Glandula parotis), Morbus Crohn (Darm), Diabetes Typ I (Bauchspeicheldrüse), Epididymitis (Epididymis), Glomerulonephritis (Nieren), Basedow'sche Erkrankung (Schilddrüse), Guillain-Barre-Syndrom (Nervenzellen), Hashimoto-Erkrankung (Schilddrüse, hämolytische Anämie (rote Blutzellen), systemischer Lupus erythematodes (mehrere Gewebe), männliche Unfruchtbarkeit (Spermium), Multiple Sklerose (Nervenzellen) Myasthenia Gravis (neuromuskuläre Synapse), Pemphigus (hauptsächlich Haut), Psoriasis (Haut), rheumatisches Fieber (Herz und Gelenke), rheumatoide Arthritis (Gelenkkapsel), Sarcoidose (mehrere Gewebe und Organe), Sklerodermie (Haut und Bindegewebe), Sjogren-Syndrom (exokrine Drüsen und andere Gewebe), Spondyloarthropathien (Axialskelett und andere Gewebe), Thyroiditis (Schilddrüse), Gefäßentzündung (Blutgefäße).

44. Verfahren zur Herstellung eines Polypeptids gemäß einem der Ansprüche 1 bis 30, umfassend:
a) Kultivieren von Wirtszellen, umfassend eine Nukleinsäure, die in der Lage ist, ein Polypeptid oder Polypeptidkonstrukt gemäß einem der Ansprüche 1 bis 30 zu kodieren, unter Bedingungen, die die Expression des Polypeptids ermöglichen, und
b) Gewinnen des hergestellten Polypeptids aus der Kultur.

45. Verfahren gemäß Anspruch 44, wobei die Wirtszellen Bakterienzellen, Hefezellen, Säugerzellen oder Insektenzellen sind.

46. Verfahren gemäß Anspruch 45, bei dem die Wirtszellen Bakterienzellen oder Hefezellen sind.

47. Verfahren gemäß Anspruch 46, bei dem die Wirtszellen E. coli-Zellen, S. cerevisiae-Zellen oder P. pastoris-Zellen sind.

48. Verfahren zum Diagnostizieren einer Störung, die durch die Dysfunktion von EGFR **gekennzeichnet** ist, umfassend die folgenden Schritte:
a) Kontaktieren einer Probe mit einem Polypeptid gemäß einem der Ansprüche 1 bis 30,
b) Detektieren einer Bindung des Polypeptids an die Probe, und
c) Vergleichen der in Schritt (b) detektieren Bindung mit einem Standard, wobei ein Unterschied im Binden relativ zur Probe eine Diagnose einer Störung ist, die durch Dysfunktion von EGFR **gekennzeichnet** ist.

49. Kit zur Untersuchung auf eine Störung, die durch Dysfunktion von EGFR **gekennzeichnet** ist, umfassend ein Polypeptid gemäß einem der Ansprüche 1 bis 30.

## Revendications

1. Polypeptide anti-EGFR comprenant au moins un anticorps simple domaine dirigé contre l'EGFR, dans lequel ledit anticorps simple domaine est dérivé d'un anticorps à chaîne lourde naturellement dépourvu de chaîne légère (VHH) et dans lequel ledit au moins un anticorps simple domaine est capable d'internaliser l'EGFR sur les cellules EGFR⁺.

2. Polypeptide anti-EGFR selon la revendication 1, pouvant être obtenu par l'incubation de phages recombinants exprimant un répertoire de VHH clonés avec des cellules EGFR⁺ dans des conditions pour permettre l'internalisation des phages et la libération ultérieure des phages internalisés.

3. Polypeptide anti-EGFR selon l'une quelconque des revendications 1 ou 2, dans lequel la cellule EGFR⁺ est choisie parmi les cellules HER-14 et A431.

4. Polypeptide anti-EGFR selon l'une quelconque des revendications 1 à 3, dans lequel le au moins un anticorps simple domaine correspond à une séquence représentée par l'une quelconque de SEQ ID No. : 21, 22 ou
a) une séquence homologue de l'une quelconque de SEQ ID No. : 21, 22 avec une identité de séquence de plus de 70 % avec la séquence parentale ;
b) une partie fonctionnelle de l'une quelconque de SEQ ID No. : 21, 22 qui conserve l'interaction avec la cible avec une affinité de 1 x 10⁻⁶ M ou mieux ;
c) une partie fonctionnelle de l'une quelconque de SEQ ID No. : 21, 22 qui comprend une délétion partielle de la séquence d'acides aminés complète et conserve toujours le(s) site(s) de liaison et le(s) domaine(s) protéique(s) nécessaire(s) à la liaison de et l'interaction avec EGFR.

5. Polypeptide anti-EGFR selon l'une quelconque des revendications 1 à 4, dans lequel le au moins un anticorps simple domaine correspond à une séquence représentée par l'une quelconque de SEQ ID No. : 21, 22, ou à une séquence homologue de l'une quelconque de SEQ ID No. : 21, 22 avec une identité de séquence de plus de 80 % avec la séquence parentale.

6. Polypeptide anti-EGFR selon l'une quelconque des revendications 1 à 5, dans lequel le au moins un anticorps simple domaine correspond à une séquence représentée par l'une quelconque de SEQ ID No. : 21, 22, ou à une séquence homologue de l'une quelconque de SEQ ID No. : 21, 22 avec une identité de séquence de plus de 90 % avec la séquence parentale.

7. Polypeptide anti-EGFR selon l'une quelconque des revendications 1 à 6, dans lequel le au moins un anticorps simple domaine concurrence ou inhibe la liaison du ligand naturel de l'EGFR.

8. Polypeptide anti-EGFR selon la revendication 7, dans lequel ledit ligand naturel est l'EGF.

9. Polypeptide anti-EGFR selon l'une quelconque des revendications 1 à 8, dans lequel le au moins un anticorps simple domaine est capable de se lier à sa cible avec une affinité meilleure que 10⁻⁶ M.

10. Polypeptide anti-EGFR selon l'une quelconque des revendications 1 à 9, comprenant en outre au moins un anticorps simple domaine dirigé contre une ou plusieurs protéines sériques.

11. Polypeptide anti-EGFR selon la revendication 10, dans lequel ledit au moins un anticorps simple domaine dirigé contre une ou plusieurs protéine(s) sérique(s) est dirigé contre l'une quelconque de la sérum albumine, les immunoglobulines sériques, la protéine liant la thyroxine, la transferrine, ou le fibrinogène ou un fragment de ceux-ci.

12. Polypeptide anti-EGFR selon l'une quelconque des revendications 1 à 11, dans lequel le au moins un anticorps simple domaine est un domaine VHH.

13. Polypeptide anti-EGFR selon la revendication 12, dans lequel le au moins un anticorps simple domaine est un domaine VHH portant un acide aminé du groupe constitué par la glycine, l'alanine, la valine, la leucine, l'isoleucine, la proline, la phénylalanine, la tyrosine, le tryptophane, la méthionine, la sérine, la thréonine, l'asparagine, ou la glutamine en position 45 selon la numérotation de Kabat.

14. Polypeptide anti-EGFR selon la revendication 12, dans lequel le au moins un anticorps simple domaine est un domaine VHH qui porte un résidu arginine en position 103 selon la numérotation de Kabat.

15. Polypeptide anti-EGFR selon l'une quelconque des revendications 12 à 14, dans lequel ledit domaine VHH est obtenu par l'immunisation d'un chameau et l'obtention d'hybridome de celui-ci, ou par le clonage d'une banque d'anticorps simple domaine après une sélection par l'utilisation d'une expression phagique.

16. Polypeptide anti-EGFR selon l'une quelconque des revendications 1 à 15, dans lequel le au moins un anticorps simple domaine est un VHH humanisé.

17. Polypeptide anti-EGFR selon la revendication 16, dans lequel le au moins un anticorps simple domaine est humanisé en remplaçant un ou plusieurs des acides aminés de *Camelidae* par leur contrepartie humaine comme trouvé dans la séquence consensus humaine.

18. Polypeptide anti-EGFR selon l'une quelconque des revendications 16 ou 17, dans lequel le au moins un anticorps simple domaine est humanisé en remplaçant l'un quelconque des résidus suivants soit seul soit en combinaison : FR1 positions 1, 5, 28 et 30, l'acide aminé sceau en position 44 et 45 dans FR2, les résidus FR3 74, 75, 76, 83, 84, 93 et 94 et les positions 103, 104, 108 et 111 dans FR4 ; en numérotant selon la numérotation de Kabat.

19. Polypeptide anti-EGFR selon l'une quelconque des revendications 1 à 18, comprenant en outre au moins un anticorps simple domaine choisi dans le groupe constitué par un anticorps simple domaine anti-IFN-gamma, un anticorps simple domaine anti-TNF-alpha, un anticorps simple domaine anti-récepteur du TNF-alpha et un anticorps simple domaine anti-récepteur de l'IFN-gamma.

20. Polypeptide anti-EGFR selon l'une quelconque des revendications 1 à 19, dans lequel le nombre d'anticorps simple domaine dirigés contre l'EGFR est au moins deux.

21. Polypeptide anti-EGFR selon la revendication 20, dans lequel les deux anticorps simple domaine ou plus sont différents en terme de séquence.

22. Polypeptide anti-EGFR selon la revendication 20, dans lequel les deux anticorps simple domaine ou plus sont identiques en terme de séquence.

23. Polypeptide anti-EGFR selon l'une quelconque des revendications 19 à 22, dans lequel les deux anticorps simple domaine ou plus sont fusionnés génétiquement au niveau de l'ADN.

24. Polypeptide anti-EGFR selon la revendication 23, dans lequel les anticorps simple domaine sont liés directement les uns aux autres.

25. Polypeptide anti-EGFR selon l'une quelconque des revendications 19 à 24, qui est une molécule bivalente, trivalente ou tétravalente.

26. Polypeptide anti-EGFR selon l'une quelconque des revendications 1 à 25, comprenant en outre un support qui améliore le transfert à travers la paroi intestinale dans la circulation sanguine.

27. Polypeptide anti-EGFR selon la revendication 26, dans lequel ledit support est un VHH qui se lie spécifiquement à un récepteur sur la paroi intestinale.

28. Polypeptide anti-EGFR selon l'une quelconque des revendications 1 à 27, comprenant en outre un support qui améliore le transport du polypeptide de la lumière pulmonaire vers le sang.

29. Polypeptide anti-EGFR selon la revendication 28, dans lequel ledit support est un VHH qui se lie spécifiquement à un récepteur sur la surface de la muqueuse.

30. Polypeptide anti-EGFR selon la revendication 29, dans lequel ledit récepteur est le récepteur Fc N (FcRn).

31. Acide nucléique codant pour un polypeptide selon l'une quelconque des revendications 1 à 30.

32. Cellule hôte comprenant l'acide nucléique selon la revendication 31.

33. Composition comprenant un polypeptide selon l'une quelconque des revendications 1 à 30 ou un acide nucléique selon la revendication 31, et un support pharmaceutique convenable.

34. Composition selon la revendication 33 adaptée à la voie d'administration choisie, c'est-à-dire, les voies orale, vaginale, rectale, parentérale, intranasale, par inhalation, sublinguale, intraveineuse, intramusculaire, topique ou sous-cutanée.

35. Composition selon l'une quelconque des revendications 33 ou 34, convenable pour une injection ou une perfusion.

36. Composition thérapeutique comprenant un polypeptide anti-EGFR selon l'une quelconque des revendications 1 à 30 en combinaison avec un agent anti-néoplasique ou chimiothérapeutique.

37. Composition thérapeutique selon la revendication 36 pour une administration séparée des composants.

38. Polypeptide anti-EGFR selon l'une quelconque des revendications 1 à 30, acide nucléique selon la revendication 31, ou composition selon l'une quelconque des revendications 33 à 37 pour une utilisation dans le traitement et/ou la prévention de troubles liés à des processus inflammatoires.

39. Polypeptide anti-EGFR selon l'une quelconque des revendications 1 à 30, acide nucléique selon la revendication 31, ou composition selon l'une quelconque des revendications 33 à 37 pour une utilisation dans le traitement et/ou la prévention des cancers épithéliaux, tels qu'un carcinome du poumon, du foie, du système nerveux central, de l'os, du sang et du système lymphatique, du côlon, du sein, de la prostate, du rectum, de la vésicule biliaire, de la tête et du cou, de l'ovaire, du testicule, pancréatique et des cellules squameuses.

40. Polypeptide anti-EGFR selon i'une quelconque des revendications 1 à 30, acide nucléique selon la revendication 31, ou composition selon l'une quelconque des revendications 33 à 37, pour une utilisation dans le traitement et/ou la prévention des maladies auto-immunes, telles que la maladie d'Addison (surrénale), les maladies auto-immunes de l'oreille (oreille), les maladies auto-immunes de l'oeil (oeil), l'hépatite auto-immune (foie), la parotidite auto-immune (glandes parotides), la maladie de Crohn (intestin), le diabète de type I (pancréas), l'épididymite (épididyme), la glomérulonéphrite (reins), la maladie de Graves (thyroïde), le syndrome de Guillain-Barré (cellules nerveuses), la maladie d'Hashimoto (thyroïde), l'anémie hémolytique (globules rouges), le lupus érythémateux systémique (multiples tissus), l'infertilité masculine (sperme), la sclérose en plaques (cellules nerveuses), la myasthénie grave (jonction neuromusculaire), le pemphigus (principalement cutané), le psoriasis (peau), le rhumatisme articulaire aigu (coeur et articulations), l'arthrite rhumatoïde (membrane synoviale), la sarcoïdose (multiples tissus et organes), la sclérodermie (peau et tissus conjonctifs), le syndrome de Sjôgren (glandes exocrines, et autres tissus), les spondylarthropathies (squelette axial, et autres tissus), la thyroïdite (thyroïde), la vasculite (vaisseaux sanguins).

41. Utilisation d'un polypeptide ou d'une construction polypeptidique selon l'une quelconque des revendications 1 à 30 ou d'un acide nucléique selon la revendication 31, pour la préparation d'un médicament destiné au traitement d'un trouble lié aux processus inflammatoires et au cancer.

42. Utilisation d'un polypeptide ou d'une construction polypeptidique selon l'une quelconque des revendications 1 à 30 ou d'un acide nucléique selon la revendication 31, pour la préparation d'un médicament destiné au traitement, et/ou à la prévention des cancers épithéliaux, tels qu'un carcinome du poumon, du foie, du système nerveux central, de l'os, du sang et du système lymphatique, du côlon, du sein, de la prostate, du rectum, de la vésicule biliaire, de la tête et du cou, de l'ovaire, du testicule, pancréatique et des cellules squameuses.

43. Utilisation d'un polypeptide ou d'une construction polypeptidique selon l'une quelconque des revendications 1 à 30 ou d'un acide nucléique selon la revendication 31, pour la préparation d'un médicament destiné au traitement, et/ou à la prévention des maladies auto-immunes, telles que la maladie d'Addison (surrénale), les maladies auto-immunes de l'oreille (oreille), les maladies auto-immunes de l'oeil (oeil), l'hépatite auto-immune (foie), la parotidite auto-immune (glandes parotides), la maladie de Crohn (intestin), le diabète de type I (pancréas), l'épididymite (épididyme), la glomérulonéphrite (reins), la maladie de Graves (thyroïde), le syndrome de Guillain-Barré (cellules nerveuses), la maladie d'Hashimoto (thyroïde), l'anémie hémolytique (globules rouges), le lupus érythémateux systémique (multiples tissus), l'infertilité masculine (sperme), la sclérose en plaques (cellules nerveuses), la myasthénie grave (jonction neuromusculaire), le pemphigus (principalement cutané), le psoriasis (peau), le rhumatisme articulaire aigu (coeur et articulations), l'arthrite rhumatoïde (membrane synoviale), la sarcoïdose (multiples tissus et organes), la sclérodermie (peau et tissus conjonctifs), le syndrome de Sjôgren (glandes exocrines, et autres tissus), les spondylarthropathies (squelette axial, et autres tissus), la thyroïdite (thyroïde), la vasculite (vaisseaux sanguins).

44. Procédé de production d'un polypeptide selon l'une quelconque des revendications 1 à 30, comprenant :
(a) la culture de cellules hôtes comprenant un acide nucléique capable de coder pour un polypeptide ou une construction polypeptidique selon l'une quelconque des revendications 1 à 30 dans des conditions permettant l'expression du polypeptide, et,
(b) la récupération du polypeptide produit à partir de la culture.

45. Procédé selon la revendication 44, dans lequel lesdites cellules hôtes sont des cellules bactériennes, des cellules de levure, des cellules de mammifère ou des cellules d'insecte.

46. Procédé selon la revendication 45, dans lequel lesdites cellules hôtes sont des cellules bactériennes ou des cellules de levure.

47. Procédé selon la revendication 46, dans lequel lesdites cellules hôtes sont des cellules de *E. coli,* des cellules de *S. cerevisiae* ou des cellules de *P. Pastoris*.

48. Procédé de diagnostic d'un trouble **caractérisé par** le dysfonctionnement de l'EGFR comprenant les étapes de :
(a) mise en contact d'un échantillon avec un polypeptide selon l'une quelconque des revendications 1 à 30,
(b) détection de la liaison dudit polypeptide audit échantillon, et
(c) comparaison de la liaison détectée à l'étape (b) avec un étalon, dans laquelle une différence dans la liaison par rapport audit échantillon est un diagnostic d'un trouble **caractérisé par** un dysfonctionnement de l'EGFR.

49. Kit pour le dépistage d'un trouble **caractérisé par** un dysfonctionnement de l'EGFR comprenant un polypeptide selon l'une quelconque des revendications 1 à 30.
